# EUROPEAN PATENT APPLICATION

(11) **EP 1 839 614 A2**
(43) Date of publication of application: **03.10.2007**
(21) Application number: 07251259.3
(22) Date of filing: 23.03.2007
(51) Int. Cl.: A61B 17/88, A61B 17/82

(54) **Kit and device for use in orthopaedic procedures**

(30) Priority: 30.03.2006 US 393507
(71) Applicant: DePuy Products, Inc., Warsaw, IN 46581 (US)
(72) Inventor: Leisinger, Steven R., Silver Lake, IN 46982 (US)
(74) Representative: Belcher, Simon James

(57) **Abstract**

A kit for use in orthopaedic procedures on a long bone includes a cable and a device. The device includes a body. The device also includes first connecting means operably associated with the body for connecting a first end of the cable to the device. The device also includes second connecting means operably associated with the body for connecting a second end of the cable to the device. The first connecting means and/or the second connecting means include a unidirectional locking mechanism for tightening the cable.

## Description

The present invention relates generally to the field of orthopaedics, and more particularly, to an implant for use in arthroplasty.

Patients who suffer from the pain and immobility caused by osteoarthritis and rheumatoid arthritis have an option of joint replacement surgery. Joint replacement surgery is quite common and enables many individuals to function properly when it would not be otherwise possible to do so. Artificial joints are usually comprised of metal, ceramic and/or plastic components that are fixed to existing bone.

Such joint replacement surgery is otherwise known as joint arthroplasty. Joint arthroplasty is a well-known surgical procedure by which a diseased and/or damaged joint is replaced with a prosthetic joint. In a typical total joint arthroplasty, the ends or distal portions of the bones adjacent to the joint are resected or a portion of the distal part of the bone is removed and the artificial joint is secured thereto.

There are known to exist many designs and methods for manufacturing implantable articles, such as bone prostheses. Such bone prostheses include components of artificial joints such as elbows, hips, knees and shoulders.

A joint within the human body forms a juncture between two or more bones or other skeletal parts. The ankle, hip, knee, shoulder, elbow and wrist are just a few examples of the multitude of joints found within the body. As should be apparent from the above list of examples of joints, many of the joints permit relative motion between the bones. For example, the motion of sliding, gliding, hinge or ball and socket movements may be had by a joint. For example, the ankle permits a hinge movement, the knee allows for a combination of gliding and hinge movements and the shoulder and hip permit movement through a ball and socket arrangement.

The joints in the body are stressed or can be damaged in a variety of ways. For example, the gradual wear and tear is imposed on the joints through the continuous use of a joint over the years. The joints that permit motion have cartilage positioned between the bones providing lubrication to the motion and also absorbing some of the forces direct to the joint. Over time, the normal use of a joint may wear down the cartilage and bring the moving bones in a direct contact with each other. In contrast, in normal use, a trauma to a joint, such as the delivery of a large force, from an accident for, example, an automobile accident, may cause considerable damage to the bones, the cartilage or to other connective tissue such as tendons or ligaments.

Arthropathy, a term referring to a disease of the joint, is another way in which a joint may become damaged. Perhaps the best known joint disease is arthritis, which is generally referred to a disease or inflammation of a joint that results in pain, swelling, stiffness, instability, and often deformity.

There are many different forms of arthritis, with osteoarthritis being the most common and resulting from the wear and tear of a cartilage within a joint. Another type of arthritis is osteonecrosis, which is caused by the death of a part of the bone due to loss of blood supply. Other types of arthritis are caused by trauma to the joint while others, such as rheumatoid arthritis, Lupus, and psoriatic arthritis destroy cartilage and are associated with the inflammation of the joint lining.

The hip joint is one of the joints that is commonly afflicted with arthropathy. The hip joint is a ball and socket joint that joins the femur or thighbone with the pelvis. The pelvis has a semispherical socket called the acetabulum for receiving a ball socket head in the femur. Both the head of the femur and the acetabulum are coated with cartilage for allowing the femur to move easily within the pelvis. Other joints commonly afflicted with arthropathy include the spine, knee, shoulder, carpals, metacarpals, and phalanges of the hand.

Arthroplasty as opposed to arthropathy commonly refers to the making of an artificial joint. In severe cases of arthritis or other forms of arthropathy, such as when pain is overwhelming or when a joint has a limited range of mobility, a partial or total replacement of the joint within an artificial joint may be justified. The procedure for replacing the joint varies, of course, with the particular joint in question, but in general involves replacing a terminal portion of an afflicted bone with a prosthetic implant and inserting a member to serve as a substitute for the cartilage.

The prosthetic implant is formed of a rigid material that becomes bonded with the bone and provides strength and rigidity to the joint and the cartilage substitute members chosen to provide lubrication to the joint and to absorb some of the compressive forces. Suitable material for the implant include metals, and composite materials such as titanium, cobalt chromium, stainless steel, ceramic and suitable materials for cartilage substitutes include polyethylene. A cement may also be used to secure the prosthetic implant to the host bone.

A total hip replacement, for example, involves removing the ball shaped head of the femur and inserting a stem implant into the centre of the bone, which is referred to as the medullary canal, or marrow of the bone. The stem implant may be cemented into the medullary canal or may have a porous coated surface for allowing the bone to heal directly to the implant. The stem implant has a neck and a ball shaped head, which are intended to perform the same functions as a healthy femur's neck and a ball shaped head. The polyethylene cup is inserted into the acetabulum and has a socket for receiving the head on the stem implant.

The stem implant of a total hip replacement is positioned in the medullary canal after the medullary canal marrow has been reamed or surgically removed from the medullary canal. The condition of the outer or cortical bone or the femur after the femur has been resected and the medullary canal reamed may vary in its thickness and strength. If the patient is particularly elderly and if the patient has osteoporotic bone, the thickness and strength of the cortical bone remaining after reaming of the medullary canal may be both thin and weak. The insertion of the stem component of the total hip prosthesis into the medullary canal may cause the cortical bone to fracture, shatter or splinter.

To accommodate such fracture, splintering or shattering of the cortical bone of the resected femur, a wire or cable may be extended circumferentially around the outer periphery of the cortical bone to secure the fragments of cortical bone to themselves and to provide support for the stem implant of the total hip prosthesis. The ends of the cable are secured by a clamp or sleeve. Such a cable is known as a cerclage cable. In fact, a plurality of spaced apart cables may be necessary to adequately secure the fractured femur and to support the stem implant.

It should be appreciated that a cable or wire may be placed circumferentially about any long bone to provide for additional support for receiving a prosthesis to reduce the chance of the cortical bone fracturing or splitting. Also, it should be appreciated that the cable or wire may be utilized for any long bone and may be placed in, for example, the shaft portion of any long bone. For example, the cable or wires may be placed on, for example, a tibia, a fibula, a femur, a humerus, an ulna, or any other long bone of the body.

It should be appreciated that a cable may be utilized as part of a trauma procedure when the bone,, particularly the shaft of a long bone, becomes fractured and, in particular, if the long bone has longitudinal fractures. The cable or wire may be used, for example, for a humoral fracture related to the fall of an elderly patient or for any femur, tibia or fibula fracture.

To install a cerclage cable or wire properly around the periphery of the shaft portion of a bone, the tension or clamping force of the cable is preferably, accurately controlled.

Prior art devices have been developed to provide for an accurate tensioning of a cerclage cable.

Various prior art attempts have addressed the issue of accurately tensioning a cerclage cable. For example, a technique is disclosed in US-6482208 and US-5595994 in which a threaded shaft is rotated to provide the tension to the cerclage cable. An alternative technique which is disclosed in US-5449361 and WO-99/09904 uses a ratchet and pawl-type mechanism to provide the tension to the cerclage cable.

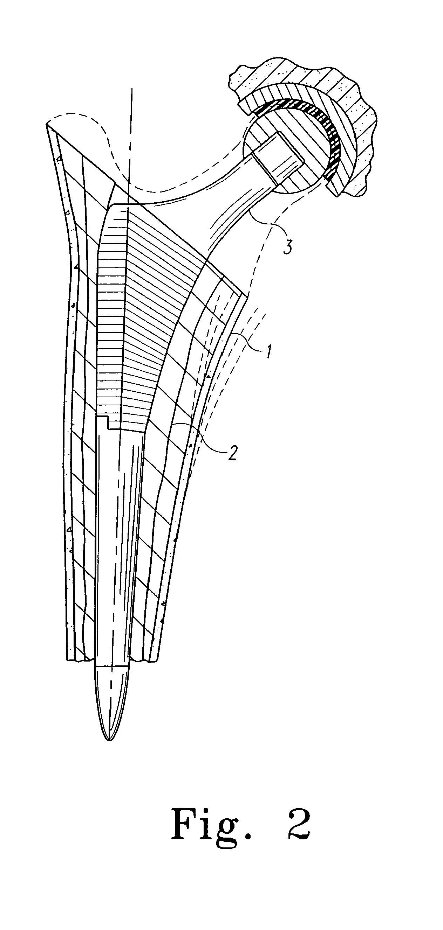

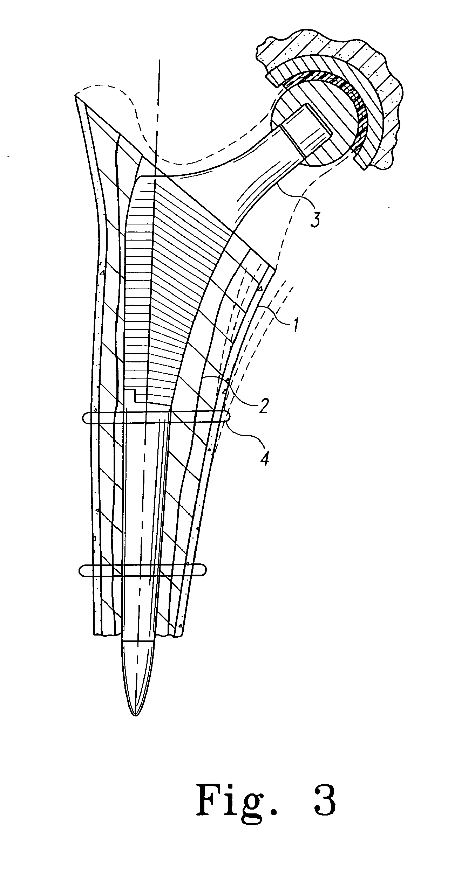

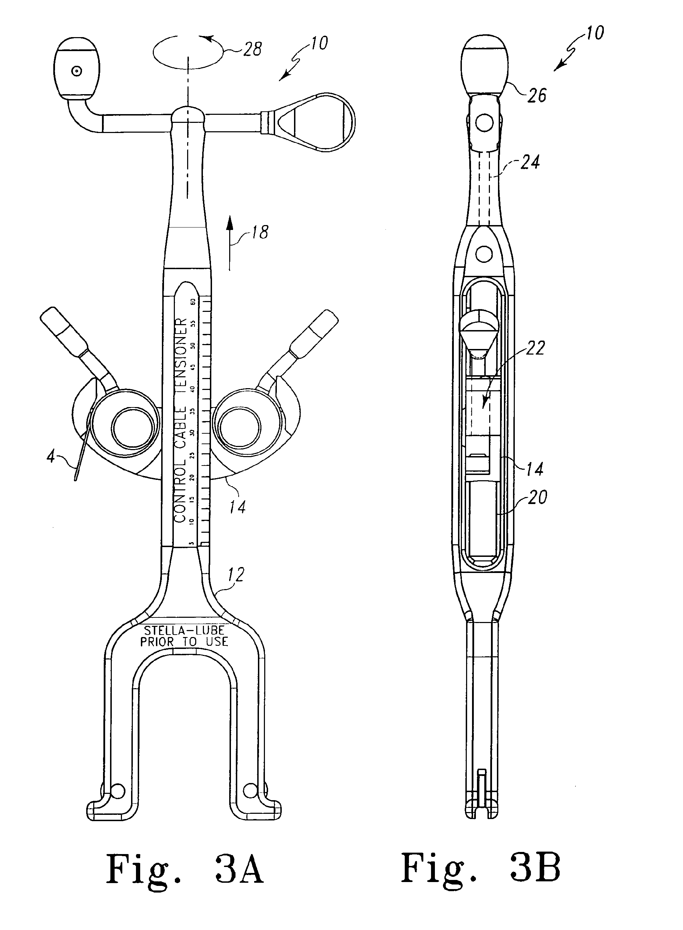

Referring now to FIGS. 2 and 3, another prior art cerclage tensioner in the form of control cable tensioner throttle no. 2739-42-000 sold by DePuy Orthopaedics, Inc. is shown.

Referring now to FIG. 2, a long bone in the form of femur 1 is shown having an axial fracture 2. The long bone 1 has been resected and a prosthesis in the form of hip stem 3 is inserted into the femur 1. It should be appreciated that the fracture 2 of the femur 1 greatly weakens the femur 1 and may serve to provide an inadequate support for the hip stem 3. It should be appreciated that a remedy is needed for the fracture 2 of the femur 1 to properly support the hip stem 3. Referring now to FIG. 3, a common remedy for the fracture 2 of the femur 1 is shown as cerclage cable 4. The cerclage cable 4 surrounds the periphery of the femur 1 and serves to contain the fractured portions of the femur 1 to provide better support for the hip stem 3. For example, and as shown in FIG. 3, a plurality of spaced apart cerclage cables 4 may be utilized for assisting the femur 1 in properly supporting the hip stem 3.

It should be appreciated that the cerclage cable 4 may be utilized to provide additional support for any fractured long bone. In particular, the cerclage cable 4 may be suited for supporting the shaft portion of any long bone so that a prosthetic member including a intramedullary stem portion may be properly supported by a fractured long bone.

Referring now to FIGS. 3A and 3B, a cerclage control cable tensioner is shown control cable tensioner 10.

Referring now to FIG. 3A, the cable tensioner 10 is shown. The cable tensioner 10 includes a body 12 and a slide 14 slidably movable with respect to the body 12. The body 12 includes an opening 16 for positioning the long bone 1. The cable 4 is connected to the cable tensioner 10. For example, the cable 4 is connected from slide 14 to the body 12 and across the opening 16. The cable 4 then is secured at its opposed end to the slide 14. The cable 4 is wrapped around the long bone 1 in the opening 16 of the body 12 and is secured to the slide 14. As the slide 14 advances in the direction of arrow 18, the cable 4 is tensioned.

Referring now to FIG. 3B, the slide 14 is slidably fitted to the body 12 by means of a track 20 formed in the body 12 and closely conforming to the slide. The slide 14 may, for example, include an internally threaded opening 22, which cooperates with an externally threaded shaft 24. The shaft 24 is connected to, for example, handle 26, which when rotated in the direction of arrow 28 advances the slide 14 in the direction of arrow 18 with respect to the body 12, thereby tightening the cable 4 around the long bone 1.

The cable tensioner 10 of FIGS. 3A and 3B is complicated and expensive to manufacture. The tensioner 10 also has a limited mechanical advantage and thus a limited maximum tension that it can provide to the cable. Further, the track 20, as well as, the threaded internal opening 22 and the externally threaded shaft 24 provide for an adjustment mechanism that may require lubrication and maintenance. The tensioner also may not provide accurate tension control.

According to the present invention, a device for tensioning a cerclage cable that provides sufficient access for attachment of the crimping mechanism is provided.

The cable-tensioning device utilizes two binding mechanisms to hold the cable ends and a ratcheting pulley for creating an accurate tension control over a wide variety of tension forces.

The cable tensioner of the present invention includes a fork-like body. The fork-like body receives a first end of the cable. The first end of the cable is secured to the body. The cable is extended over the opening of the fork-like body and is positioned around the long bone. The second end of the cable is connected to a ratcheting mechanism. The ratcheting mechanism is used to tension the cable. Once the cable is properly tensioned, the cable is secured with, for example, a crimping procedure. The ratcheting mechanism may be integral with a drive pulley and may be rotated to tension the cable.

According to an aspect of the present invention, a device for use in tensioning a cable around a long bone for use in orthopaedic procedures is provided. The device includes a body. The device also includes first connecting means operably associated with the body for connecting a first end of the cable to the device. The device also includes second connecting means operably associated with the body for connecting a second end of the cable to the device. The first connecting means and/or the second connecting means include a unidirectional locking mechanism for tightening the cable.

According to another aspect of the present invention, a kit for use in orthopaedic procedures on a long bone is provided. The kit includes a cable and a device. The device has a base and first and second spaced-apart arms extending from the base. The first and second arms each include a closed end attached to the base and an open end opposed to the closed end. The device also includes first connecting means. The first connecting means is connected to the first arm for connecting a first end of the cable to the device. The device further includes second connecting means connected to the second arm for connecting a second end of the cable to the device. The first connecting means includes a unidirectional locking mechanism for tightening the cable.

The kit and device of the invention can be used in a method for combining bone fragments on a long bone of a patient is provided. The method includes the steps of providing a device including a body having a base and first and second spaced-apart arms extending from the base. The device has a tensioning device connected to the second arm. The method also includes the steps of providing a cable, securing the cable to the first arm of the device, wrapping the cable around the long bone, securing the cable to the second arm of the device, and tensioning the cable with the tensioning device.

According to yet another aspect of the present invention, a kit for use in orthopaedic procedures on a long bone is provided. The kit includes a cable and a device. The device includes a body. The device also includes first connecting means operably associated with the body for connecting a first end of the cable to the device. The device also includes second connecting means operably associated with the body for connecting a second end of the cable to the device. The first connecting means and/or the second connecting means include a unidirectional locking mechanism for tightening the cable.

According to yet another aspect of the present invention, a device for use in tensioning a cable around a long bone for use in orthopaedic procedures is provided. The device includes a base and a first spaced-apart arm extending from the base. The first arm includes a closed end attached to the base and an open end opposed to the closed end. The device also includes a second spaced-apart arm extending from the base. The second arm includes a closed end attached to the base and an open end opposed to the closed end. The device also includes first connecting means. The first connecting means is connected to the first arm for connecting a first end of the cable to the device. The first connecting means includes a unidirectional locking mechanism for tightening the cable. The first connecting means is operably associated with the body or the first arm for connecting a first end of the cable to the device. The device also includes second connecting means connected to the second arm for connecting a second end of the cable to the device.

According to another aspect of the present invention, a method for combining bone fragments on a long bone of a patient is provided. The method includes the step of providing a device including a body having a base and first and second spaced-apart arms extending from the base. The arms have proximal and distal ends of the arms and have a tensioning device associated with the second arm. The method also includes the steps of providing a cable and securing the cable to the distal end of the first arm of the device. The method includes the step of wrapping the cable around the long bone, securing the cable to the distal end of the second arm of the device, and tensioning the cable with the tensioning device.

The technical advantages of the present invention include the ability of the tensioning device to not seize during its use. For example, according to one aspect of the present invention, a device for use in tensioning a cable around a long bone is provided. The device includes a body having a base and first and second spaced apart arms. The device also includes a first connecting means and a second connecting means with a cable being positioned between the first connecting means the second connecting means. The device provides for a low friction guiding of the cable between the first connecting means the second connecting means. Thus, the present invention provides for a tensioning device for a cable, in which the cable does not seize.

The technical advantages of the present invention further include the ability to provide broader control of final tension and greater maximum tension for a tensioning of a cerclage cable. For example, according to another aspect of the present invention, a device for use in tensioning a cable around a long bone is provided. The device includes a body having first and second spaced apart arms and a connecting mean having a unidirectional locking mechanism for tightening the cable. The unilateral locking mechanism may include a low friction mechanism, such as a ratcheting mechanism that will provide for broad tension control and greater maximum tension. Thus, the present invention provides for a tensioning device with much broader control of final tension and greater maximum tension force.

The technical advantages of the present invention further include the ability to provide a orthopaedic cable-tensioning device with a simple mechanism. For example, according to another aspect of the present invention, a device for use in tensioning a cable around a long bone is provided. The device includes a body having a base and spaced apart arms extending from the base. The device further includes a first connecting means to connect a first end of the cable and a second connecting means for connecting the second end of the cable. The second connecting means includes a unidirectional locking mechanism for tightening the cable. The tightening mechanism may be a simple ratcheting mechanism and may, for example, be a pair of spring-biassed wheels including teeth for providing the unidirectional locking mechanism. Thus the present invention provides for a cable-tensioning device with a simple mechanism.

The technical advantages of the present invention further include the ability to provide a tensioning device that is easy to manufacture and maintain. For example, according to another aspect of the present invention, a device for use in tensioning a cable around a long bone is provided. The device includes a body having spaced apart arms extending from the body. A connecting means is attached to one of the arms while a second connecting means is attached to the other arm. The second connecting mean may include a unilateral locking mechanism to tighten the cable. The unilateral locking mechanism may include a ratcheting mechanism in the form of a spring-biassed pair of wheels including teeth for providing the ratcheting mechanism. This mechanism is easy to manufacture and simple to maintain. Thus, the present invention provides for a orthopaedic cable-tensioning device that is easy to manufacture and maintain.

The technical advantages of the present invention further include the ability to quickly and easily tension a cable around a long bone. For example, according to yet another aspect of the present invention, a device for use in tensioning a cable around a long bone is provided. The device includes a body with a pair of spaced apart arms extending from the base of the body. A first connecting means is used to secure one end of the cable while a second connecting means is used to secure and tighten the cable to the device. The second connecting means includes a unilateral locking mechanism in the form of, for example, a pair of spaced apart wheels having teeth, which are biassed to provide for a unilateral locking mechanism. The unilateral locking mechanism is quickly and easily secured by means of a simple ratchet mechanism that is quick and easy to use. Thus, the present invention provides for a cable-tensioning device that is quick and easy to use.

The technical advantages of the present invention further include the ability to provide a cable-tensioning device with reduced components. For example, according to yet another aspect of the present invention, a tensioning device for tensioning a cable around a long bone is provided, including a body having an integral first connecting means in the form of, for example, an opening in the body for receiving a first end of a cable and a second connecting means in the form of a ratcheting mechanism. The ratcheting mechanism may include simply three components, a first wheel, a fastener to secure the first wheel to the body, and a spring for biassing the first wheel to the body. The body and the first wheel may include teeth to provide for the unilateral ratcheting mechanism. Thus the present invention provides for a tensioning device with a minimal number or a reduced amount of components.

Embodiments of the invention will now be described by way of example with reference to the accompanying drawings, in which:
FIG. 1 is a plan view of an embodiment of a tensioning device in accordance with the present invention;
FIG. 1A is a perspective view of a crimper for use with the tensioning device of the present invention;
FIG. 1B is a partial enlarged plan view of a first arm of the tensioning device of FIG. 1;
FIG. 1C is a partial end view of the arm of FIG. 1B;
FIG. 1D is a partial end view of a second arm of the tensioning device of FIG. 1;
FIG. 1E is a plan view of the body of the tensioning device of FIG. 1;
FIG. 1F is a cross-sectional view of FIG. 1 along the line 1E-1F in the direction of the arrows;
FIG. 1G is an end view of the body of FIG. 1E;
FIG. 1H is a plan view of the ratchet stem of the locking mechanism of the tensioning device of FIG. 1;
FIG. 1I is an end view of the ratchet stem of FIG. 1H;
FIG. 1J is a plan view of a pulley of the tensioning device of FIG. 1;
FIG. 1K is a plan view of a pawl of the locking mechanism of the tensioning device of FIG. 1;
FIG. 1L is an end view of the pawl of FIG. 1K;
FIG. 1 M is a plan view of the spring of the locking mechanism of the tensioning device of FIG. 1;
FIG. 1N is a partial plan view of the tensioning device of FIG. 1 showing the sleeve in greater detail;
FIG. 1O is a perspective view of the sleeve of FIG. 1N with the cable installed into the sleeve;
FIG. 1P is a perspective view of the sleeve of FIG. 1N;
FIG. 1Q is a plan view of the sleeve of FIG. 1N with the cable installed into the sleeve;
FIG. 1R is a plan view of a socket wrench for use with the tensioning device of FIG. 1;
FIG. 1 S is a plan view of a beam torque wrench for use with the tensioning device of FIG. 1;
FIG. 1T is a plan view of a break away torque wrench for use with the tensioning device of FIG. 1;
FIG. 1U is a plan view of a power wrench for use with the tensioning device of FIG. 1;
FIG. 1V is a plan view of a power wrench with torque limiter for use with the tensioning device of FIG. 1;
FIG. 1W is a perspective view of the jaws of the crimper of FIG. 1A;
FIG. 1X is a plan view of a pair of diagonal pliers for use in cutting the cable of FIG. 1O;
FIG. 1Y is a plan view of a hook for use in pulling the cable of FIG. 1O;
FIG. 2 is a plan view of a hip stem positioned in a longitudinally fractured femur;
FIG. 3 is a plan view of a hip stem positioned in a longitudinally fractured femur with a cable cerclage positioned over the outer periphery of the femur;
FIG. 3A is a plan view of a prior art cable tensioner;
FIG. 3B is an end view of the prior art cable tensioner of FIG. 2;
FIG. 4 is a plan view of another embodiment of a tensioning device according to the present invention;
FIG. 4A is a partial plan view of the first arm of the tensioning device of FIG. 4 showing the pulley in the load position;
FIG. 4B is a partial plan view of the first arm of the tensioning device of FIG. 4 showing the pulley in the locked position;
FIG. 4C is a partial end view of the first arm of the tensioning device of FIG. 4;
FIG. 4D is a partial plan view of the second arm of the tensioning device of FIG. 4 showing the second pulley in greater detail;
FIG. 4E is a partial end view of FIG. 4D;
FIG. 4F is a partial end view of the second arm of the tensioning device of FIG. 4 showing the gear train in greater detail;
FIG. 4G is a partial end view of the second arm of the tensioning device of FIG. 4 showing the locking mechanism in greater detail;
FIG. 5 is a plan view of yet another embodiment of a tensioning device according to the present invention;
FIG. 6 is a partial plan view of the first arm of the tensioning device of FIG. 5;
FIG. 6A is a partial end view of the first arm of the tensioning device of FIG. 5;
FIG. 7 is a partial end view of the locking mechanism of the tensioning device of FIG. 5 showing the locking mechanism in greater detail;
FIG. 8 a partial plan view of the locking mechanism of the tensioning device of FIG. 5;
FIG. 9 is a plan view of the guide pulley of the first arm of the tensioning device of FIG. 5;
FIG. 10 is a plan view of the ratcheting stem of the tensioning device of FIG. 5;
FIG. 11 is a partial plan view of the ratcheting stem of FIG. 10 showing the teeth of the stem in greater detail;
FIG. 12 is a partial plan view of the teeth of the stem in engagement with the teeth of the arm of the tensioning device of FIG. 5;
FIG. 13 is a partial plan view of the teeth of the wheel in contact with the teeth of the arm of the cable tensioning device of FIG. 5 to permit ratcheting;
FIG. 14 is a plan view of a spring for use in the tensioning spool of FIG. 4;
FIG. 15 is a plan view of another embodiment of a tensioning device in accordance with the present invention having a spool on the first arm of the tensioning device;
FIG. 16 is a plan view of another embodiment of a tensioning device in accordance with the present invention having a spool on the end of the second arm of the tensioning device and the locking mechanism positioned on the base of the tensioning device;
FIG. 17 is a plan view of another embodiment of a tensioning device in accordance with the present invention having a spool on the end of each arm of the tensioning device;
FIG. 18 is a plan view of another embodiment of a tensioning device in accordance with the present invention having a spool on the end of each arm of the tensioning device and the locking mechanism positioned on the base of the tensioning device;
FIG. 19 is a plan view of yet another embodiment of a tensioning device in accordance with the present invention having a spool on the end of each arm of the tensioning device and the locking mechanism positioned on an arm of the tensioning device;
FIG. 20 is a plan view of another embodiment of a tensioning device in accordance with the present invention having a spool on the end of each arm of the tensioning device and another embodiment of a locking mechanism positioned on an arm of the tensioning device;
FIG. 21 is a plan view of a kit for performing joint arthroplasty including the tensioning device of the present invention;
FIG. 22 is a process flow diagram of a method of performing joint arthroplasty surgery in accordance with yet another embodiment of the present;
FIG. 23 is a process flow diagram for a method of performing joint arthroplasty surgery according to a further embodiment of the present invention;
FIG. 24 is a plan view partially in cross section of a hip implant in use with a cable positioned over a femur that may be tensioned with the cable tensioner of the present invention;
FIG. 25 is a partial perspective view of the cable of FIG. 1Q showing the cable in position on a long bone; and
FIG. 26 is a partial plan view of the cable of FIG. 1Q showing the cable connection in greater detail.

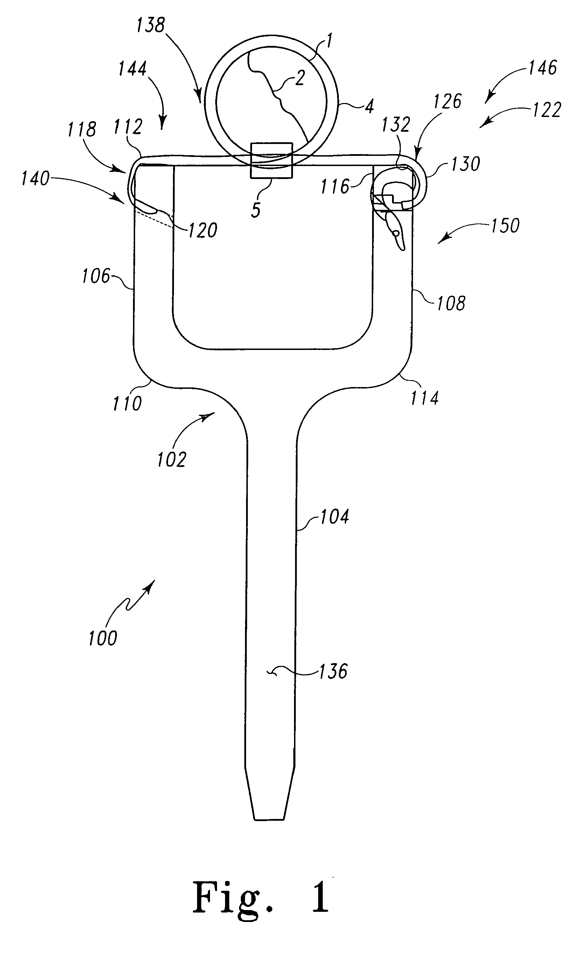

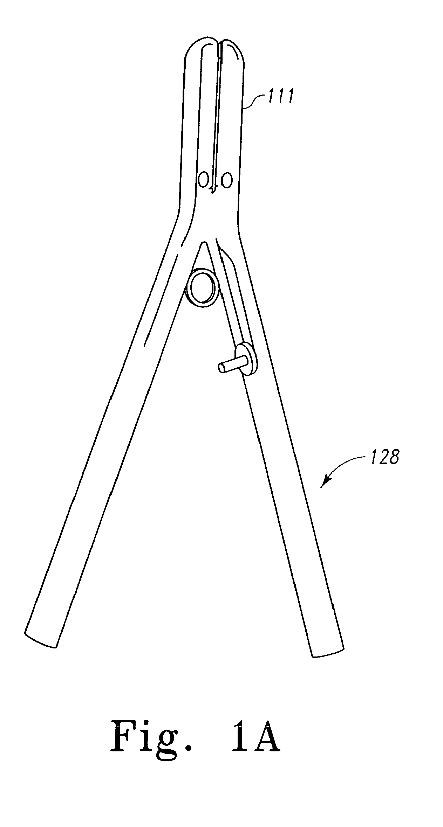

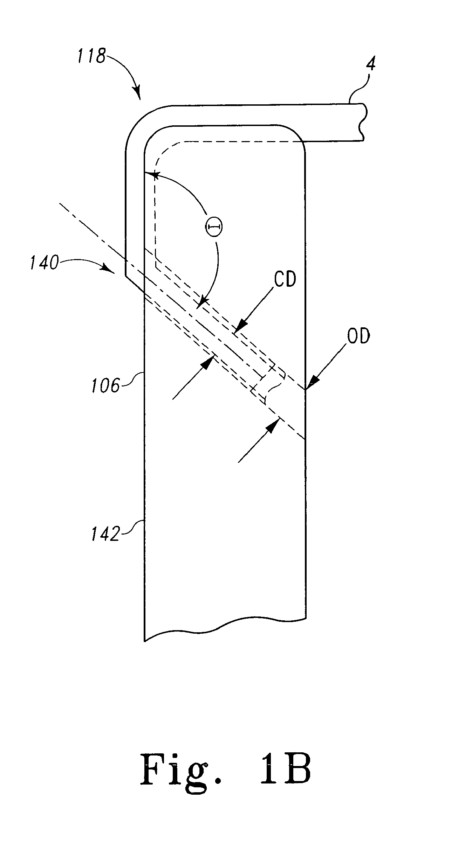

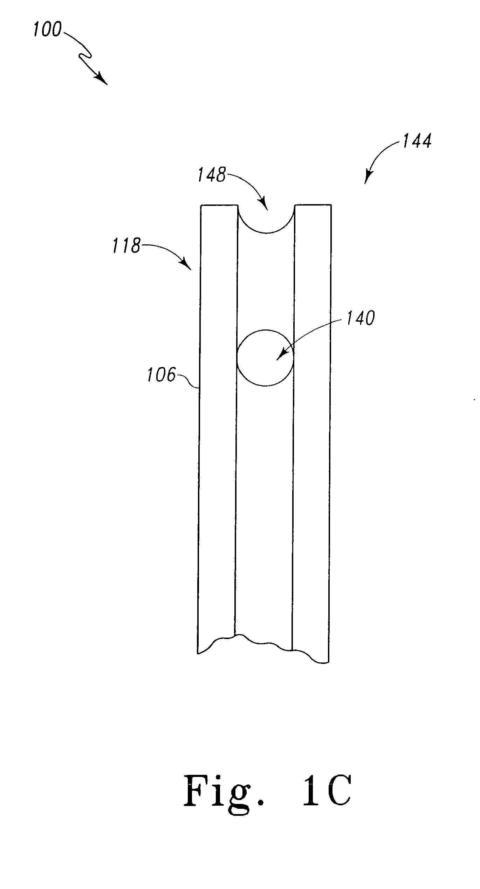

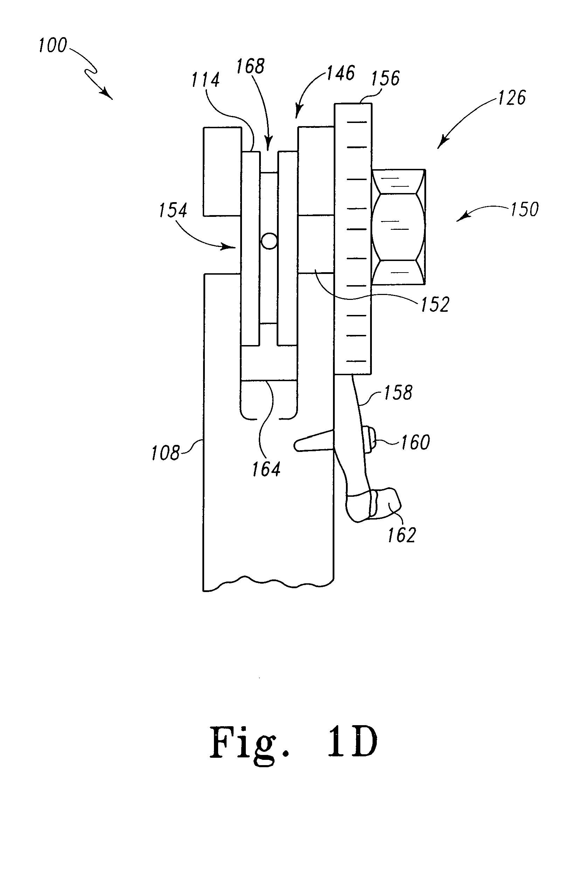

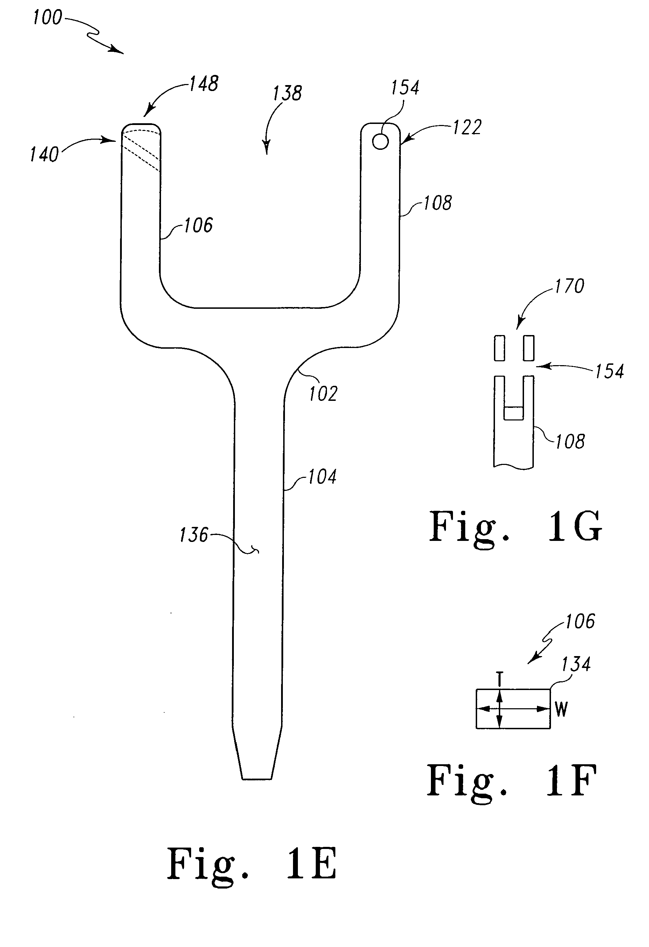

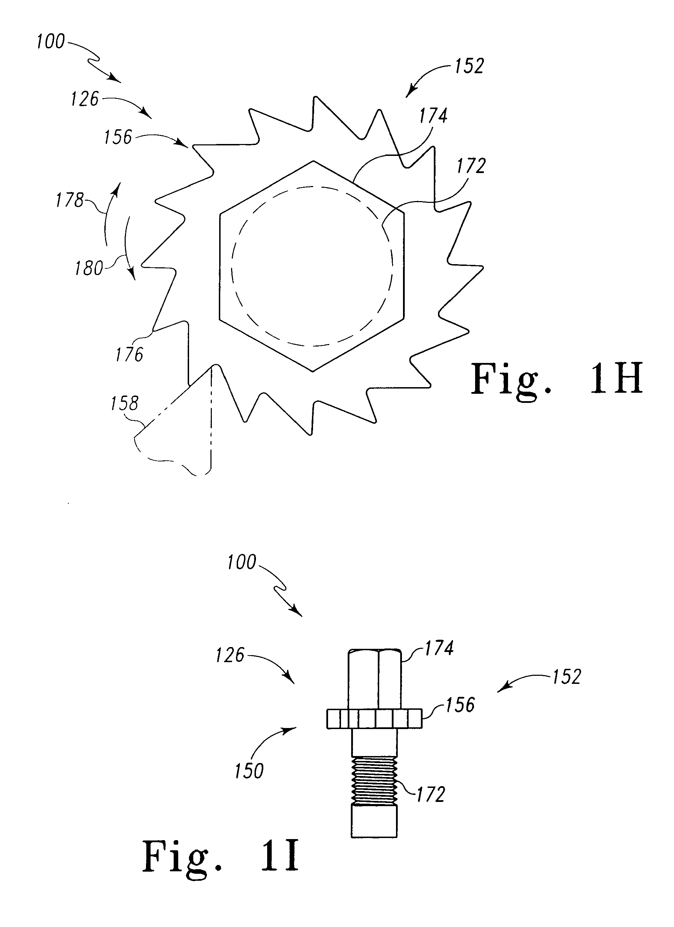

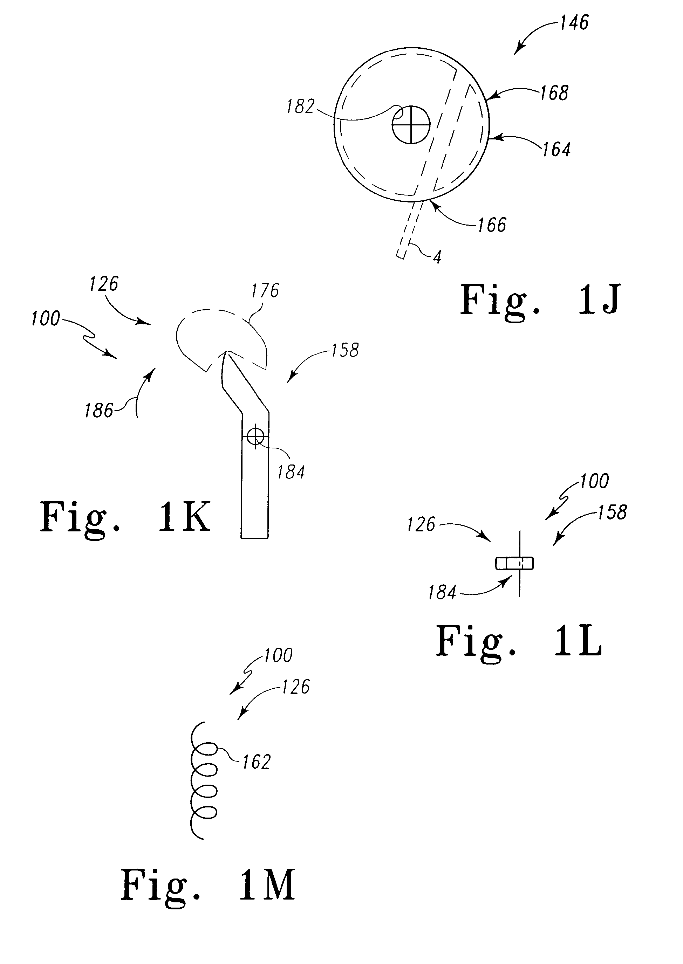

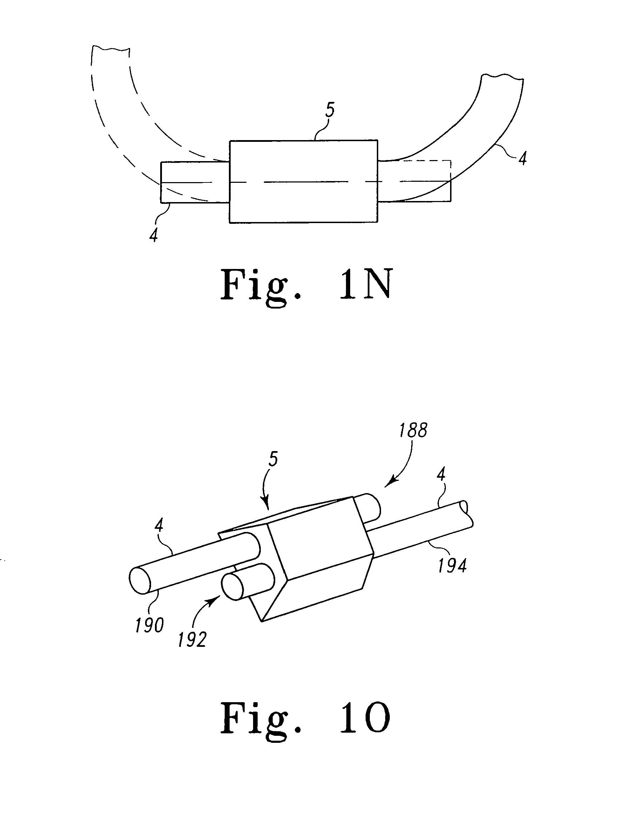

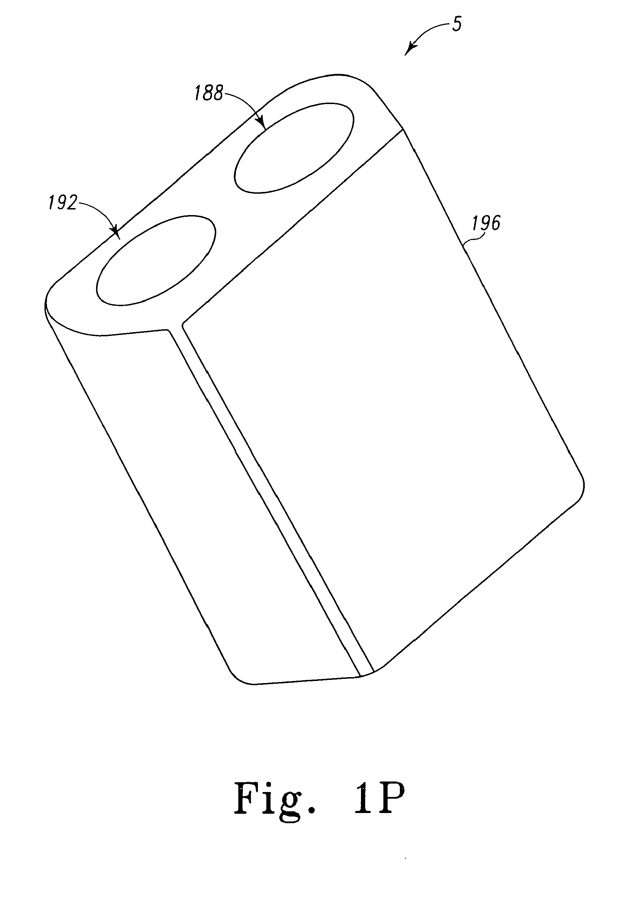

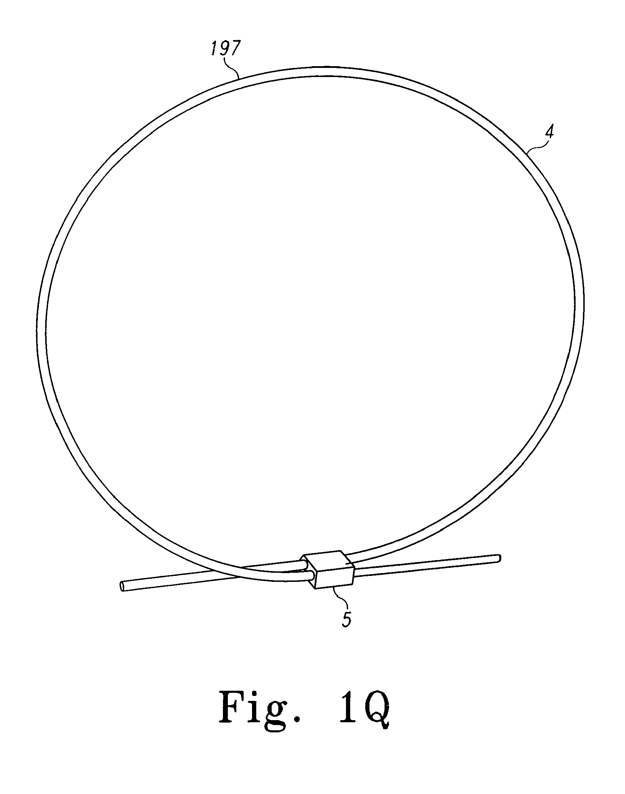

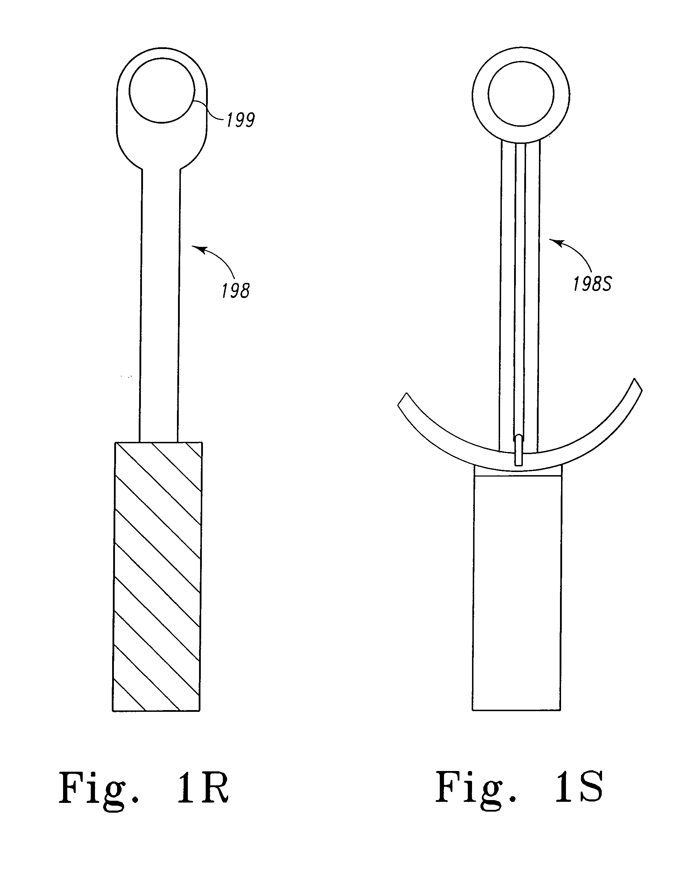

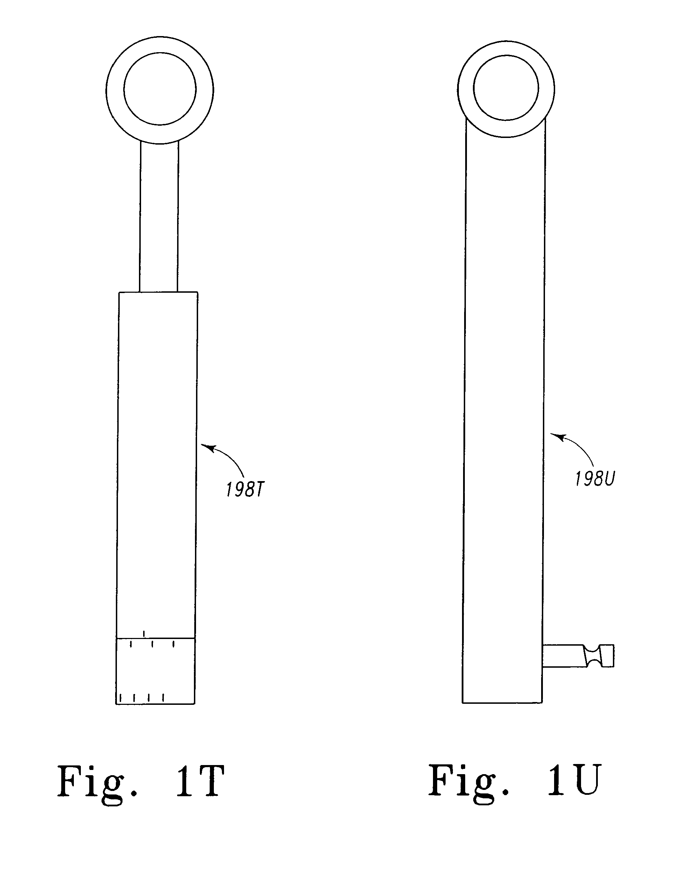

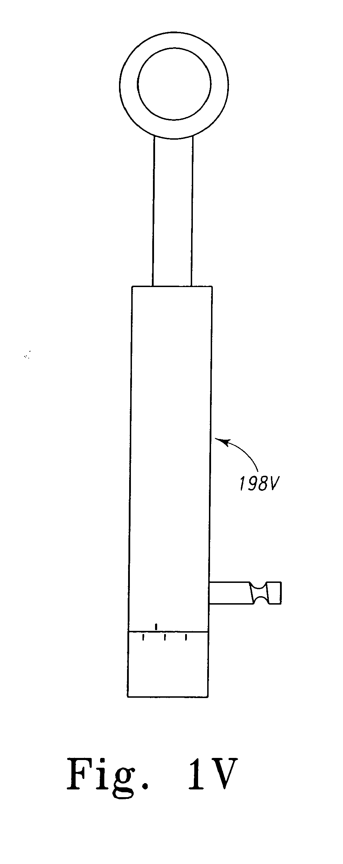

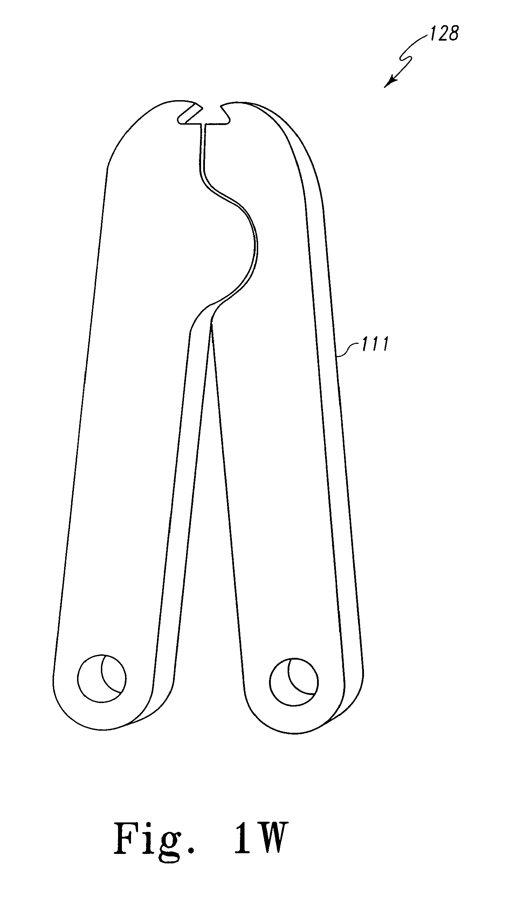

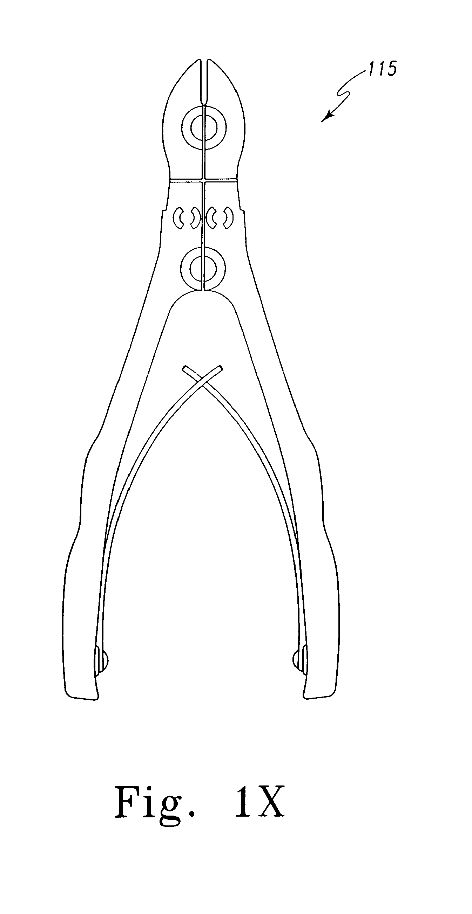

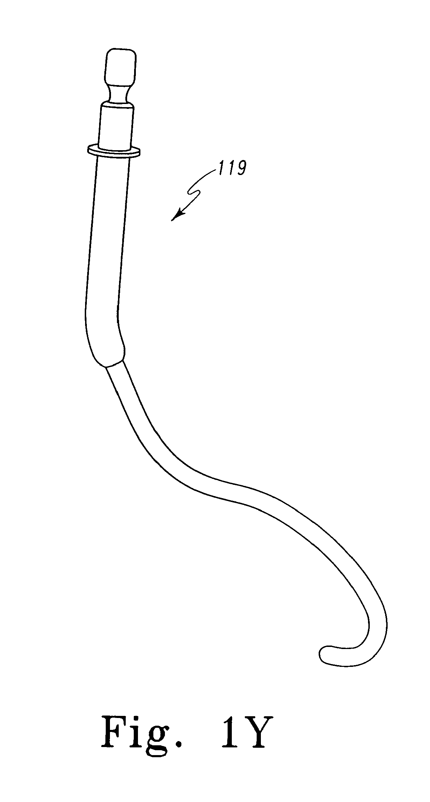

Referring to the drawings, FIG. 1 shows a device 100 for use in tensioning a cable 4 around long bone 1 for use in orthopaedic procedures. The device 100 includes a body 102 having a base 104. A first arm 106 and a second arm 108 extend from the base 104 of the body 102 of the device 100. The first arm 106 includes a closed end 110 attached to the base 104 and an open end 112 opposed to the closed end 110. Similarly, the second arm 108 includes a closed end 114 attached to the base 104 and an open end 116 opposed to the closed end 114. The device 100 further includes first connecting means 118 operably associated with the body 102 for connecting a first end 120 of the cable 4 to the device 100.

The device 100 further includes second connecting means 122 operably associated with the body 102 for connecting a second end 124 of the cable 4 to the device 100. Either first connecting means 118 or second connecting means 122, or both, include a unidirectional locking mechanism 126 for tightening the cable 4 around long bone or femur 1.

The first arm 106 and the second arm 108 may have any suitable shape or configuration. For example, and as shown in FIG. 1, the first arm 106 and the second arm 108 may extend in a substantially parallel spaced apart relationship. Further, the first arm 106 and the second arm 108 may have any suitable shape. For example, and as shown in FIG. 1, the first arm 106 may have a generally rectangular cross section.

For example and as show in FIG. 1F, the first arm 106 may have a width W as well as a thickness T. Similarly, the second arm 108 may have the width W as well as thickness T.

It should be appreciated that the first arm 106 and the second arm 108 may have similar or different cross sections. Further, it must be appreciated that the first arm 106 and the second arm 108 may have any suitable cross section such as a circular, an elliptical, or a polygon cross section.

The first arm 106 may be spaced apart from the second arm 108 in an amount sufficient to permit the cable 4 to be secured after the device 100 is used to tension the cable 4. After the cable 4 is tensioned, a crimp or sleeve 5 is utilized to secure the cable 4 to the long bone 1. The crimp or sleeve 5 is compressed by a crimper 128 (see FIG. 1D). For example, the first arm 106 may be separated from the second arm 108 a distance G to allow ample space for the use of the crimper 128 as is shown in FIG. 1D. Referring again to FIG. 1, open ends 112 and 116 of the arms 106 and 108, respectively, may include features to avoid damage to soft tissues in the form of arcuate surfaces 130 that may be in a semi-cylindrical form defined by radii extending from origin 132 a distance, for example, of radius R. The arcuate surfaces may minimize damage to soft tissue when the device 100 is used. It should be appreciated that the corners 134 of the first arm 106 and the second arm 108 may likewise include radii or chamfers to minimize damage to soft tissue.

As shown in FIG. 1, preferably the arms 106 and 108 extend beyond the base 104 to accommodate the crimping of crimp or sleeve 5 by the crimper 128 (see FIG. 1D). For example, the open end 112 of the first arm 106 and the open end 116 of the second arm 108 extend outwardly from the base 104 a distance G. The distance G is preferably sufficient for the effective use of the crimper 128 (see FIG. 1D).

Referring again to FIG. 1, the base 104 may have any suitable shape and may, for example, have a periphery 136 adapted for gripping by a health care professional's hands. For example, the periphery 136 may have a circular, oval, rectangular or polygon cross section. It may include features to assist in grasping the device 100. For example, the periphery 136 may include groove (not shown) for cooperation with the fingers. Alternatively, the periphery 136 may include knurls (not shown) or other surface roughening features to assist in grasping the device 100.

The base 104 may, as is shown in FIG. 1, be integral with the first arm 106 and the second arm 108. Alternatively, the arms 106 and 108 may be modular or include separate pieces, which are secured to the base 104 to form the body 102.

First connecting means 118 and second connecting means 122 may be positioned anywhere in operable association with the body 102. For example, and as shown in FIG. 1, the first connecting means 118 may be connected to the first arm 106. Similarly, second connecting means 122 may be positioned on the second arm 108. By positioning first connecting means 118 on the first arm 106 and second connecting means 122 on the second arm 108, the cable 4 may be strung across opening 138 positioned between the open end 112 of the first arm 108 to the open end 116 of the second arm 108.

First connecting means 118 and second connecting means 122 may have any suitable shape or be in any suitable form capable of performing the connecting function. For example, and as shown in FIG. 1, the first connecting means 118 may be defined by an opening 140 formed in the first arm 106. The opening 140 serves to slidably receive the cable 4.

Referring now to FIG. 1B, the first connecting means 118 is shown in greater detail. While the cable 4 may be mechanically secured to the first arm 106 by, for example, an interference fit, welding or soldering, or by a mechanical interlock. Alternatively, and for simplicity, however, and as shown in FIG. 1B, the cable 4 may define a diameter CD, which is slightly smaller than the diameter OD of the opening 140 formed in the first arm 106. The opening 140 may form an angle θ with outer face 142 of the first arm 106 such that as the cable 4 is tightened, the cable 4 is locked or secured to the first arm 106 at outer face 142 of first arm 106.

Referring again to FIG. 1, the cable 4 is connected from the first connecting means 118, across the opening 138, around the long bone 1, and to the second arm 108. The cable 4 is then connected to second connecting means 122. As shown in FIG. 1, the device 100 may include features for guiding the location of the cable 4 as it passes from the first arm 106 to the second arm 108. For example, and as shown in FIG. 1, the first arm 106 may include a first guiding means 144 positioned at the open end 112 of the first arm 106. Similarly, the second arm 108 may include second guiding means 146 for guiding the cable 4 and positioned open end 116 of the second arm 108.

Referring again to FIG. 1B, the cable 4 as it is strung from the first opening 140 across open end 112 of the first arm 106 is guided in the first guiding means 144. First guiding means 144 may have any shape or configuration capable of guiding the cable 4. For example, and as shown in FIG. 1B, the first guiding means 144 may be in the form of a groove 148. The groove 148 has a shape to closely conform with the cable 4.

Referring now to FIG. 1C, the first arm 106 of the device 100 is shown in an end view with first guiding means 144 in the form of the groove 148 being shown in greater detail. The groove 148 is in alignment with the opening 140.

Referring again to FIG. 1, the cable 4 is strung across the opening 138 and is secured to the second arm 108. The cable 4 is secured to the second arm 108 in any suitable fashion and may include second guiding means 146 to guide the cable 4 to the second arm 108. The device 100 further includes the second connecting means 122 for connecting the cable 4 to the device 100. The device 100 further includes the locking mechanism 126, which is adapted to lock or tautly secure the cable 4 to the device 100. Second guiding means 146, second connecting means 122 and the locking mechanism 126 may have any suitable shape and form, and may as shown in FIG. 1 be part of a second arm securing system 150.

Referring now to FIG. 1D, the second arm securing system 150 is shown in greater detail. As shown in FIG. 1D, the second arm securing system 150 includes a ratchet stem 152, which is rotatably fitted into second armhole 154 formed in the second arm 108 of the device 100. The ratchet stem 152 includes a ratchet 156 formed on the ratchet stem 152. The ratchet 156 cooperates with a pawl 158, which is pivotally secured to second arm 108 by pivot pin 160. The pawl 158 is secured to engagement with the ratchet 156 by an urging means in the form of, for example, spring 162.

A guide pulley 164 is fitted to the ratchet stem 152 and includes a guide pulley cable opening 166 for slidably receiving the cable 4. The guide pulley 164 further includes a guide pulley groove 168 for guiding the cable 4.

Referring now to FIG. 1E, the body 102 of the device 100 is shown in greater detail. The body 102 may include the base 104 as well as the first arm 106 and the second arm 108. The first arm 106 may include the opening 140 as well as the first arm groove 148. The opening 138 is formed between the first arm 106 and the second arm 108. The second arm 108 includes the second armhole 154 for receiving the second arm securing system 150 (see FIG. 1D).

The base 104, as well as the first arm 106 and the second arm 108 of the body 102, may have any suitable shape. For example, and as shown in FIG. 1F, the first arm 106 may have a generally rectangular shape which is defined by a width W and a thickness T of the first arm 106. As shown in FIG. 1F and FIG. 1, the first arm 106 may, for example, have a uniform cross section. It should be appreciated that the cross section of the first arm 106 may be circular, oval, or have a polygon shape other than a rectangular shape, for example, a triangular or pentagonal shape.

Referring now to FIG. 1G, the second arm 108 is shown in greater detail. The second arm 108 includes the second armhole 154 for cooperation with ratchet stem 152 (see FIG. 1D). The second arm 108 also includes a second arm slot 170 for receiving guide pulley 164 (see FIG. 1D).

Referring now to FIGS. 1H and 1I, ratchet stem 152 of the locking mechanism 126 of the second arm securing system 150 of the device 100 is shown in greater detail. The ratchet stem 152 includes the ratchet 156, which is positioned between external spline 172 and hexagonal head 174. The ratchet 156 includes a plurality of spaced apart teeth 176. The teeth 176 are arranged to provide for rotation of the ratchet stem 152 in the direction of arrow 178 while prohibiting rotation of the ratchet stem 152 in the direction of arrow 180.

Referring now to FIG. 1J, the guide pulley 164 is shown in greater detail. The guide pulley 164 includes a guide pulley cable opening 166 for receiving the cable 4. The guide pulley 164 further includes a guide pulley groove 168 located around the periphery of the guide pulley 164 for guiding the cable 4. The guide pulley 164 further includes a central internal spline 182 for cooperation with external spline 172 of the ratchet stem 152 (see FIG. 1H).

Referring now to FIGS. 1K and 1L, the pawl 158 of the locking mechanism 126 of the device 100 is shown in greater detail. The pawl 158 includes a pivot hole 184 for cooperation with pivot pin 160 to pivotally position the pawl 158 on the second arm 108 of the device 100 (see FIG. 1D). The spring 162 as shown in phantom engages the pawl 158 to cause the pawl 158 to rotate in the direction of arrow 186 for engagement with teeth 176 of ratchet 156 as shown in phantom.

Referring now to FIG. 1M, spring 162 is shown in greater detail. The spring 162 is utilized to urge the pawl 158 in engagement with the ratchet 156 of the ratchet stem 152 of the locking mechanism 126 of the device 100. The spring 162 may be any suitable spring and may as shown in FIG. 1M be in the form of a coil spring.

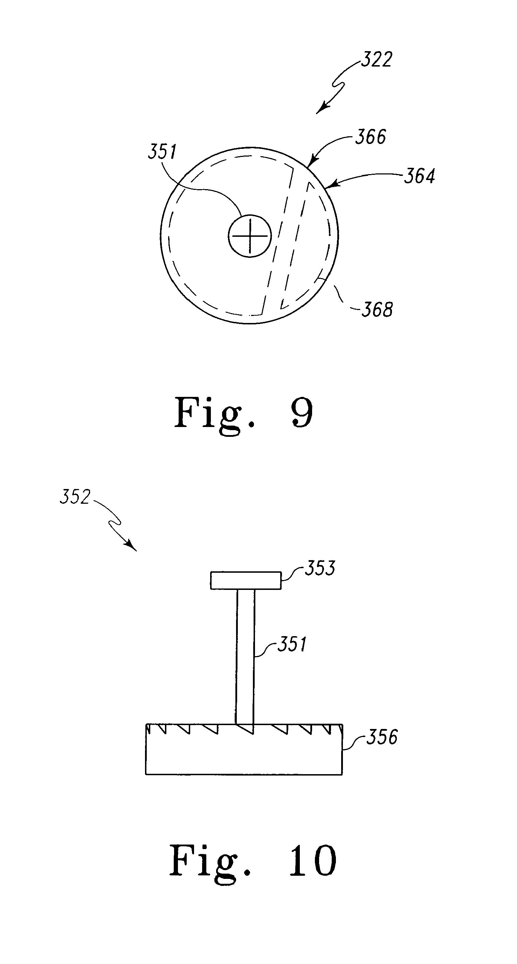

Referring now to FIGS. 1N, 10 and 1P, the sleeve 5 for use with the cable 4 in connection with the device 100 is shown in greater detail. The sleeve 5 as shown in FIG. 1O, includes a first opening 188 for receiving first portion 190 of the cable 4. The sleeve 5 further includes a second opening 192 for receiving second portion 194 of the cable 4.

Referring now to FIG. 1P, the sleeve 5 may have any suitable shape and may, as shown in FIG. 1P, have a generally rectangular shape. The sleeve 5 may include a body 196 in to which the first opening 188, and the second opening 192 are formed.

Referring now to FIG. 1Q, the cable 4 is shown in cooperation with the sleeve 5 to form cerclage cable system 197.

Referring now to FIG. 1R, a socket wrench 198 for use with the device 100 is shown. The socket wrench 198 includes an internal socket 199, which matingly fits with hexhead 174 of the ratchet stem 152 (see FIG. 1H).

Referring now to FIG. 1S, a beam torque wrench 198S is shown. The beam torque wrench 198S is similar to the socket wrench 198 of FIG. 1R, but includes a torque fork-measuring feature to control the torque, which will control the tension or force on the cable 4.

Alternatively, and as shown in FIG. 1T, a breakaway torque wrench 198T is shown. The breakaway torque wrench 198T is similar to the socket wrench 198 of FIG. 1R, except that the breakaway torque wrench 198T includes a feature to provide a specified amount of torque at which the wrench will break away or no longer be able to increase the torque force that the torque wrench will apply.

Referring now to FIG. 1U, yet another method of tightening the device 100 is shown as power wrench 198U. The power wrench 198U is similar to the socket wrench 198 of FIG. 1R, except that the power wrench 198U is used in connection with a power source. Such a power source may be, for example, a battery, an electrical circuit, a pneumatic power source or a hydraulic power source.

Referring now to FIG. 1V, yet another device for use with the device 100 to tighten the cable 4 is shown as a power wrench 198V with torque limiter. The power wrench 198V is similar to the power wrench 198U of FIG. 1U, except that the power wrench 198V has a torque-limiting feature to limit the amount of torque that the power wrench may apply to the cable 4.

The sleeve 5 may be made of any suitable durable material that may be collapsed and used to secure the cable 4. For example, the sleeve 5 may be made of a soft ductile metal. The sleeve 5 may be crimped by, for example, crimper 128A of FIG. 1A.

Referring now to FIG. 1W, the crimper 128 may include crimper inserts 111 to be used in the crimper 128 to secure the sleeve 5 to the cable 4.

Referring now to FIG. 1X, diagonal pliers 115 may be utilized to cut the cable 4 after the crimper 128 of FIG. 1A is utilized to crimp the sleeve 5 to the cable 4.

Referring now to FIG. 1Y, a hook 119 may be utilized to thread cable 4 around long bone 1.

Referring again to FIG. 1, the device 100 may be made of any suitable durable material which can be sterilised by commercially available sterilization techniques. For example, the device 100 may be made of a component made of, for example, plastics, composites or metals. For example, the body 102 may be made of, for example, a composite, a plastic or a metal. If made of a metal, the body 102 may be made of, for example, a cobalt chromium alloy, a stainless steel alloy or a titanium alloy.

The ratchet stem 152 of the device 100 may be made of, for example, a plastic, a composite or a metal. Similarly, the pawl 158 and the spring 162, and the guide pulley 164 may be made of, for example, a plastic, a composite or a metal.

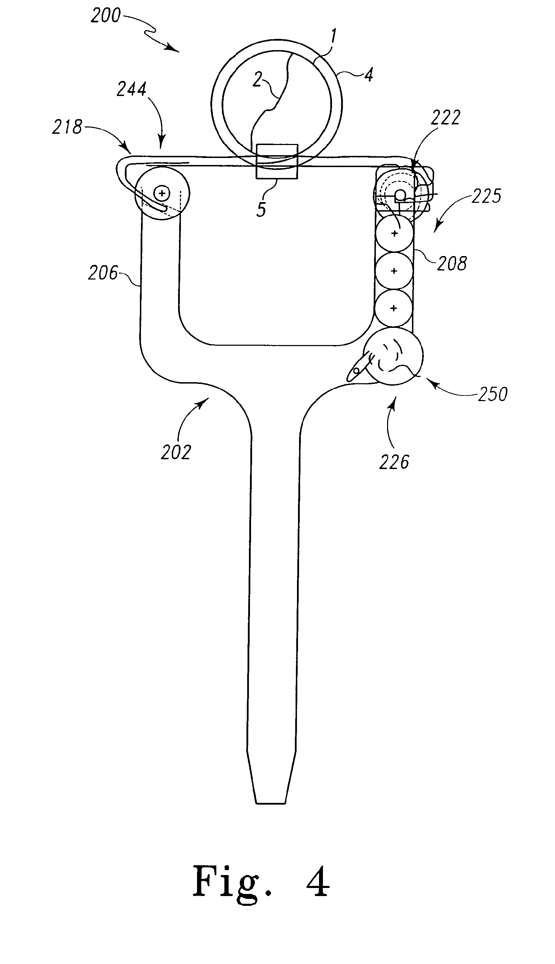

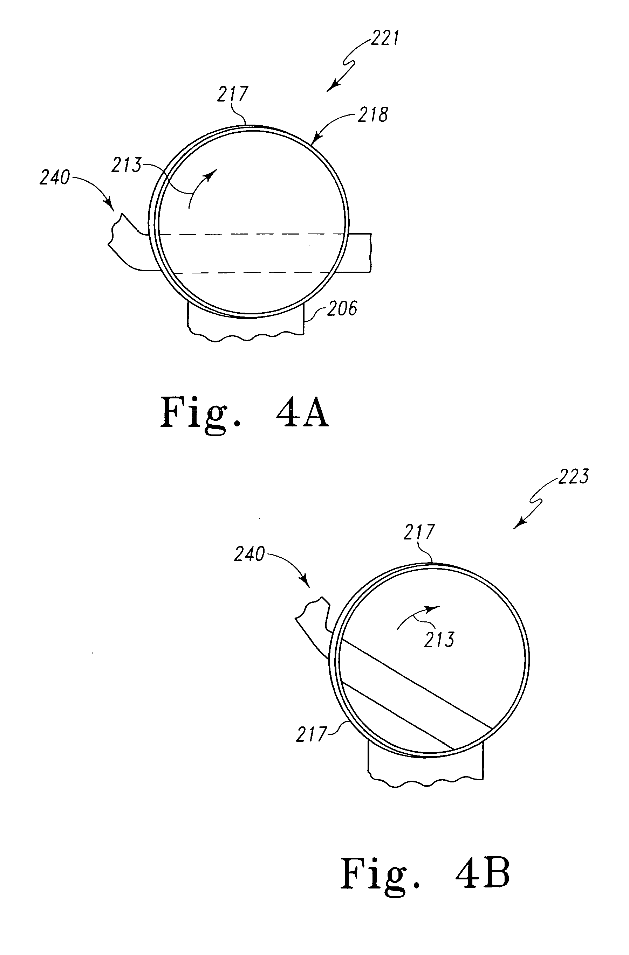

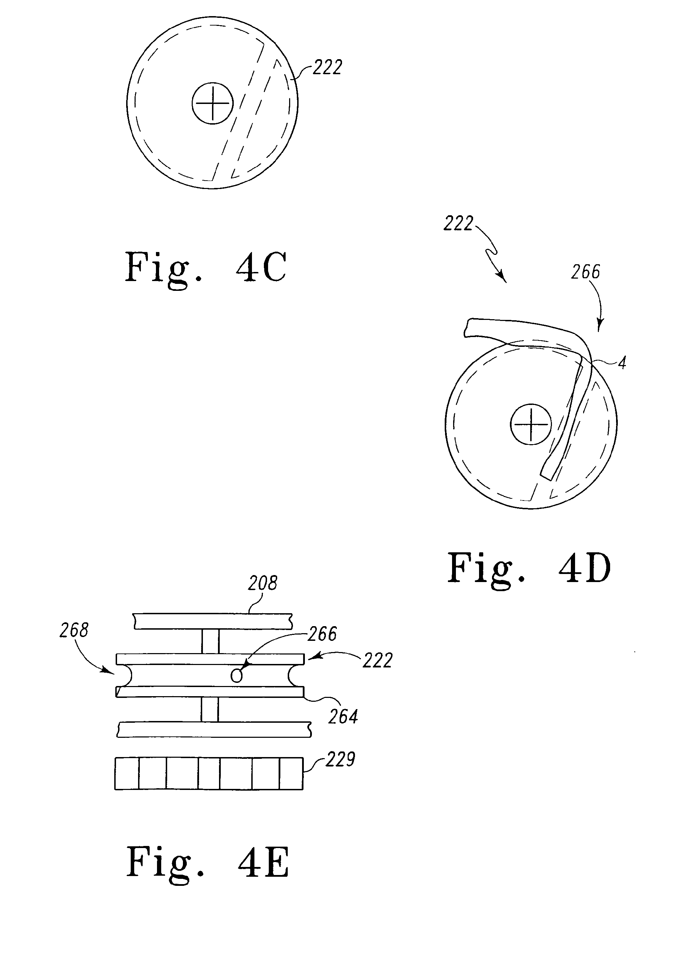

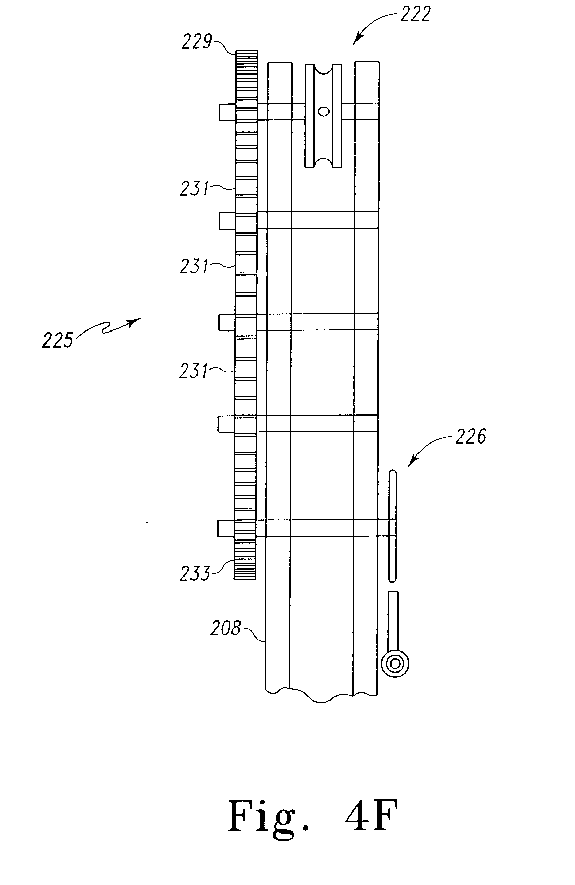

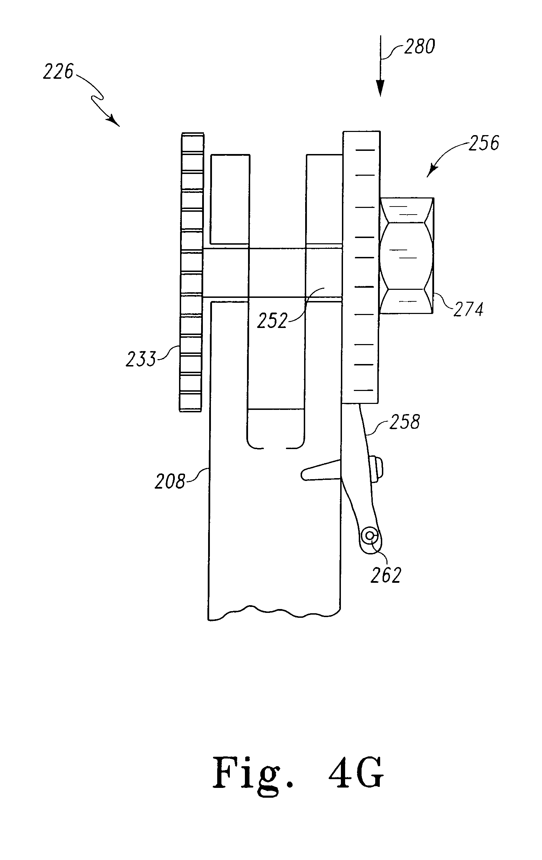

Referring now to FIG. 4, yet another embodiment of the present invention is shown as device 200. The device 200 is similar to the device 100 of FIG. 1, except that the device 200 is adapted to be used in less invasive surgeries where, for example, the incision length is less. For use in surgeries where the incision length is less, the device 200 of FIG. 4 is adapted to provide for access to tightening the cable in a position spaced further from the long bone 1 and above skin surface 7 of the patient.

For example, and as shown in FIG. 4, the device 200 includes a second arm securing system 250, which includes a locking mechanism 226, which is positioned above the skin surface 7 of the patient. The device 200 includes a body 202, including a first arm 206 and a second arm 208. A cable 4 is strung between the first arm 206 and the second arm 208. Cable 4 is connected to the first arm 206 by first connecting means 218 and to the second arm 208 by second connecting means 222.

The first connecting means 218 may be similar to the first connecting means 118 of the device 100 of FIG. 1. Alternatively, and as shown in FIG. 4, first connecting means 218 may include a first arm pulley 217, which is rotatably secured to first arm 206. The first arm pulley 217 includes an opening 240 for receiving the cable 4.

The first arm pulley 217 has a first position 221 as shown in FIG. 4A, which permits the cable 4 to be inserted into the opening 240. After the cable 4 is inserted into the opening 240, the first arm pulley 217 is rotated in the direction of arrow 213 as shown in FIG. 4A. As shown in FIG. 4A, the cable 4 is inserted into opening 240 and the first arm pulley 217 is rotated in the direction of arrow 213.

Referring now to FIG. 4B, as the first arm pulley 217 is rotated in the direction of arrow 213, the cable 4 impinges between the first arm pulley 217 and the first arm 206, locking the cable 4 in position.

Referring again to FIG. 4, the second arm securing system 250 of the device 200 is shown. The second arm securing system 250 includes the second connecting means 222. The second connecting means 222 is similar to the second connecting means 122 of the device 100 of FIG. 1. The second connecting means 222 serves to secure the opposite end of the cable 4 to the device 200. The second arm securing system 250 further includes locking mechanism 226. The locking mechanism 226 is similar to the locking mechanism 126 of the device 100, except that the locking mechanism 226 is spaced from the second connecting means 222 so that the locking mechanism 226 may be accessed at a position exterior to the skin 7 of the patient. To provide for a locking mechanism 226, which is spaced from the second connecting means 222, a gear train 225 is mechanically positioned between the second connecting means 222 and the locking mechanism 226.

Referring now to FIGS. 4D and 4E, the second connecting means 222 is shown in greater detail. The second connecting means 222 includes a guide pulley 264. The guide pulley 264 includes a cable opening 266 for receiving the cable 4. The guide pulley 264 further includes a guide pulley groove 268 for cooperation with the cable 4.

Referring now to FIG. 4F, the gear train 225 is shown in greater detail. The gear train 225 includes a drive gear 229, which is connected to the guide pulley 264. A series of intermediate gears 231 are operably connected to the drive gear 229. A driven gear 233 is connected to the intermediate gear 231. The drive gear 229, the intermediate gears 231 and the driven gear 233 are rotatably secured to the second arm 208 of the body 202 of the device 200.

Referring now to FIG. 4G, the locking mechanism 226 is shown in greater detail. The locking mechanism 226 of FIG. 4G is similar to the locking mechanism 126 of FIG. 1D. The locking mechanism 226 includes a ratchet stem 252 to which ratchet 256 is positioned. The ratchet stem 252 further includes a drive head 274 for operating the locking mechanism 226. The drive gear 233 is fixably secured to the ratchet stem 252 and rotates therewith. The ratchet stem 252 is rotatably secured to second arm 208 of the body 202 of the device 200. The ratchet 256 is engaged with a pawl 258, which permits unilateral rotation of the ratchet stem 252 in the direction of arrow 280. The pawl 258 is biassed by spring 262 to provide for the unilateral rotation in the direction of arrow 280.

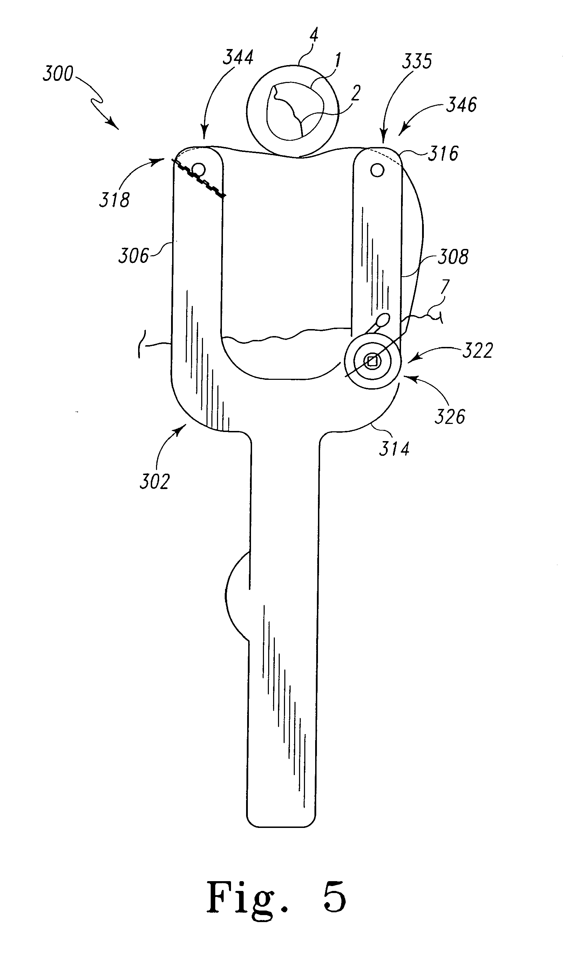

Referring now to FIG. 5, yet another embodiment of the present invention is shown as device 300. The device 300 of FIG. 5 is similar to the device 100 of FIG. 1, except that the device 300 of FIG. 5 includes a locking mechanism 326, which unlike locking mechanism 126 of FIG. 1 is positioned spaced from open end 316 of second arm 308 and positioned above skin surface 7 of the patient. The positioning of locking mechanism 326 above the skin surface 7 provides for a cable 5 that is considerably longer in length than the cable 4 of the device 100 of FIG. 1.

The device 300 of FIG. 5 is also different from the device 100 of FIG. 1, in that the locking mechanism 326 is of a different configuration than the locking mechanism 126 of the device 100. The locking mechanism 326 has a face contact ratchet and pawl locking mechanism rather than an end contact ratchet and pawl mechanism as that in the locking mechanism 126 of the device 100 of FIG. 1.

The device 300 of FIG. 5 includes a body 302 similar to the body 102 of the device 100 of FIG. 1. The body 302 includes a first arm 306 and a spaced apart parallel second arm 308. The first arm 306 includes first connecting means 318 for securing an end of the cable 4. First connecting means 318 may be similar to first connecting means 118 of the device 100 of FIG. 1.

The device 300 further includes first guiding means 344 similar to first guiding means 144 of the device 100 of FIG. 1. The device 300 further includes second guiding means 346 operably associated with open end 316 of the second arm 308. Second guiding means 346 may be similar to first guiding means 344 of the device 300 and may include a groove 335, which receives the cable 4. The device 300 further includes second connecting means 322 as well as locking means 326. Second connecting means 322 and locking means 326 are positioned near closed end 314 of the second arm 308 of the device 300.

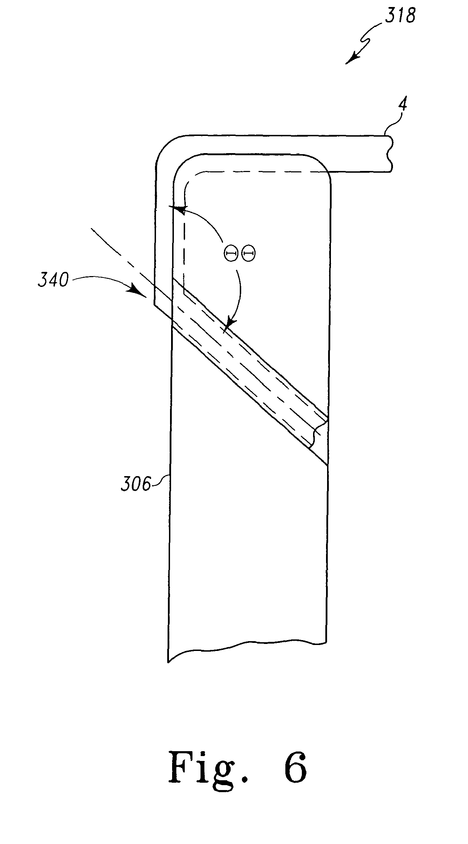

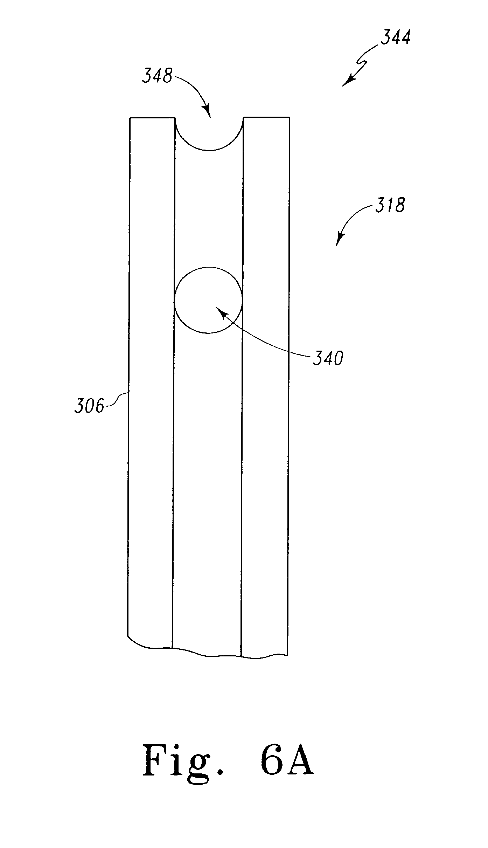

Referring now to FIGS. 6 and 6A, first connecting means 318 and first guiding means 344 are shown in greater detail. First connecting means 318 as shown in FIG. 6 includes an opening 340 for receiving the cable 4. The opening 340 is positioned at an angle θθ with respect to first arm 306.

Referring now to FIG. 6A, first guiding means 344 includes a groove 348, which matingly receives a portion of the cable 4.

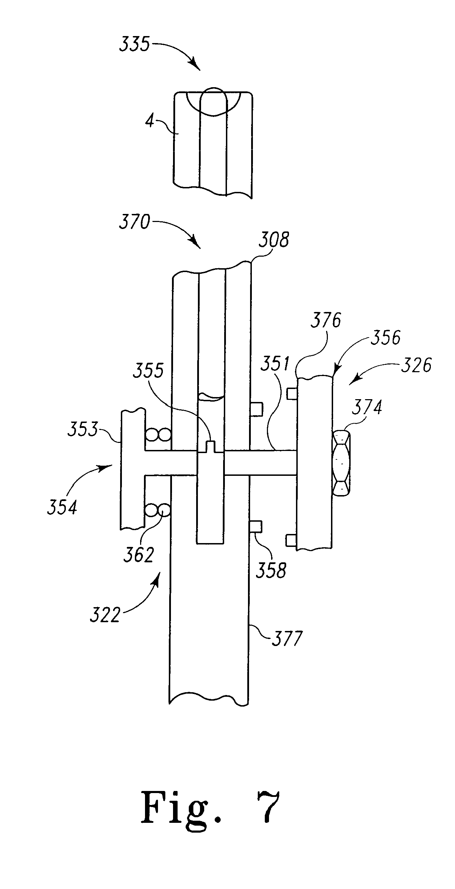

Referring now to FIG. 7, the locking mechanism 326 and second connecting means 322 are shown in greater detail. The locking mechanism 326 includes a stem 351, which is rotatably fitted in opening 354 formed in second arm 308. A ratchet 356 is fixably mounted to stem 351 and rotates therewith. A hex head 374 is secured to the ratchet 356 and is utilized to drive or rotate the locking mechanism 326. A spring 362 is positioned between the second arm 308 and spring stop 353 to urge the ratchet 356 into engagement with pawl 358 formed on second arm 308. The pawl 358 engages with a plurality spaced apart teeth 376 formed on the ratchet 356. A key 355 is positioned between the stem 351 and second connecting means 322 to cause the second connecting means 322 to rotate with the stem 351. The teeth 376 extend outwardly from face 377 of the ratchet 356 and engage the pawl 358, thereby rotating second connecting means 322 with the hex head 374. Second connecting means 322 is, as shown in FIG. 7, positioned in second arm slot 370 formed in the second arm 308.

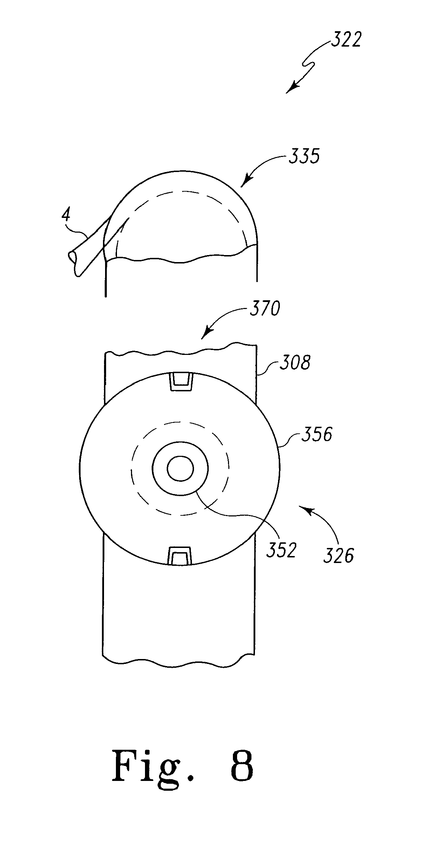

Referring now to FIG. 8, the cable 4 passes over groove 335 formed in second guide means 372 formed in second arm 308 and secured to second connecting means 322. Second connecting means 322 is rotatably connected to ratchet 356 of the locking mechanism 326.

Referring now to FIG. 9, second connecting means 322 is shown in greater detail. Second connecting means 322 includes a cylindrical guide pulley 364 defining an exterior guide pulley groove 368 for receiving the cable 4. A cable pulley opening 366 receives the cable 4 and secures the cable 4 to the guide pulley 364. The guide pulley 364 rotates about stem 351.

Referring now to FIG. 10, ratchet stem 352 is shown in greater detail. Ratchet stem 352 includes stem 351 to which stop 353 and ratchet 356 are secured.

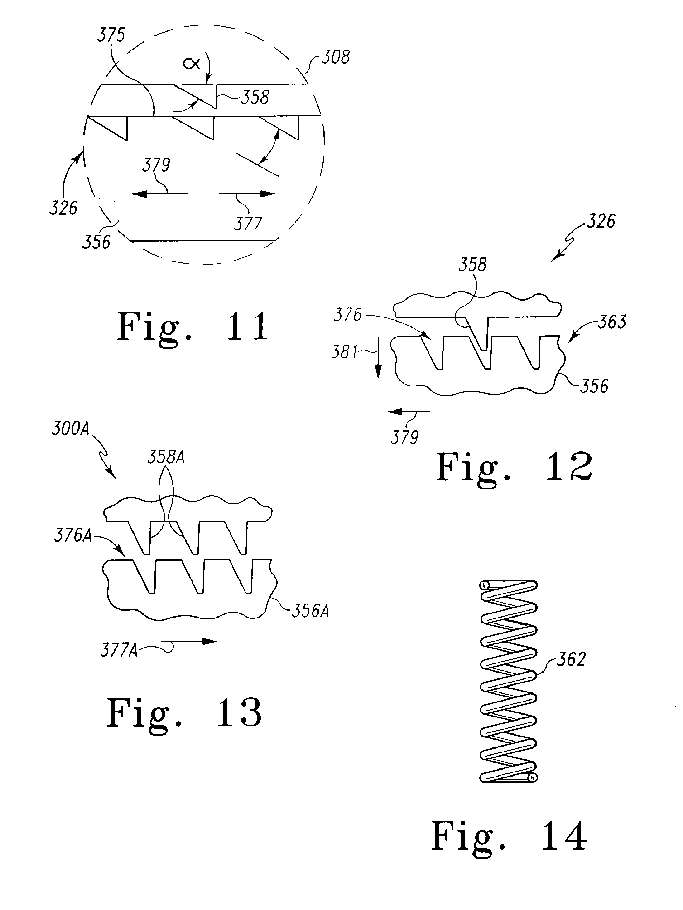

Referring now to FIG. 11, the ratchet 356 is shown in a spaced apart position with respect to the second arm 308. It should be appreciated in first position 361 as shown in FIG. 11, the ratchet 356 may rotate both in the direction of arrow 379 as well as the reverse arrow 377 with respect to the second arm 308. The second arm 308 includes a pawl 358 that may engage with teeth 376 formed in the ratchet 356. A plurality of pawls 358 may be used or a singular pawl 358 as shown may be sufficient. The teeth 376 form an angle θθθ with respect to face 375 of the ratchet 356. It should be appreciated that the pawl 358 may also form an angle α with respect to the second arm 308 whereby the angle α and the angle θθθ are similar.

Referring now to FIG. 12, the locking mechanism 326 is shown with the ratchet 356 engaged with the pawl 358. In the position as shown in FIG. 12, the ratchet 356 is prevented from moving in the direction of arrow 379, such that the ratchet 356 and the cable 4 that is attached to the ratchet 356 may be secured. The pawl 358 engages with teeth 376 formed in the ratchet 356. In second position 363, the ratchet 356 is prohibited from moving in the direction of arrow 379 and may move in the direction of arrow 379 only when the ratchet 356 moves in a direction away from the second arm 308 in a direction of arrow 381.

Referring now to FIG. 13, yet another embodiment of the present invention is shown as device 300A. The device 300A is similar to device 300 of FIG. 5, except that the device 300A includes a locking mechanism 326A that is somewhat different than the locking mechanism 326 of the device 300 of FIG. 5. The locking mechanism 326A of the device 300A includes a plurality of spaced apart pawls or external teeth 358A, which engage with internal teeth 376A formed in the ratchet 356A. The external teeth 358A formed in the second arm 308A and the internal teeth 376A formed in the ratchet 356A are biassed in engagement with each other and permit motion in the direction of arrow 377A when the ratchet 356 is spaced from the second arm 308 and prohibit motion in the direction of arrow 379A.

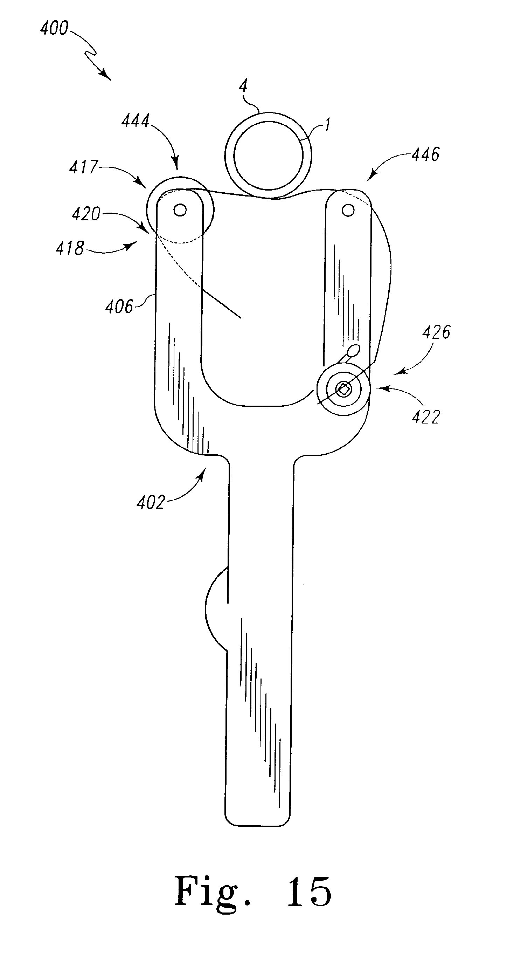

Referring now to FIG. 14, a spring 362 for use in the locking mechanism 326 of the device 300 of FIG. 5 is shown. The spring 362, as is shown in FIG. 14, is a helical spring and may be made of a metal. For example, the spring 326 may be made of a stainless steel material that is sterilizable.

It should be appreciated that the components of the device 300 of FIGS. 5 through 14 may be made of any suitable durable material that is sterilizable by a commercially available sterilization technique, for example, by an Autoclave^{™} process.

Referring now to FIG. 15, yet another embodiment of the present invention is shown as device 400. The device 400 is similar to device 300 of FIG. 5, except that the device 400 includes first connecting means 418, which is similar to first connecting means 118 of the device 100 of FIG. 1. First connecting means 418 is in the form of an opening 420 for receiving the cable 4. The device 400 further includes first guiding member 444, which is similar to the first guiding member 244 of the device 200 of FIG. 4. The first guiding member 444 of the device 400 is in the form of a pulley 417, which has an external groove for receiving the cable 4. The device 400 further includes second guiding means 446 similar to second guiding means 346 of the device 300 of FIG. 5. The device 400 further includes second connecting means 422 similar to second connecting means 322 of the device 300 of FIG. 5. The device 400 further includes a locking mechanism 426 similar to the locking mechanism 326 of the device 300 of FIG. 5.

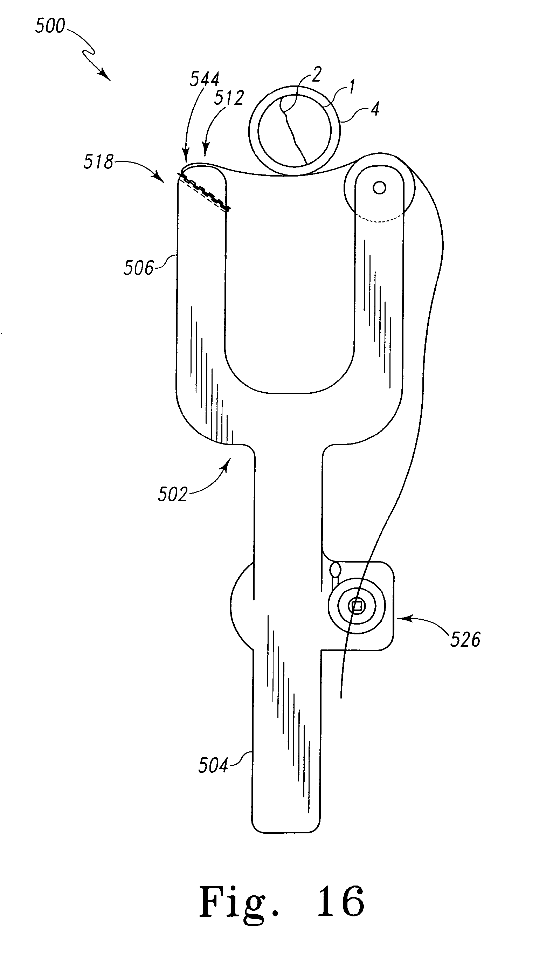

Referring now to FIG. 16, yet another embodiment of the present invention is shown as device 500. The device 500 is similar to the device 400 of FIG. 15, except that the device 500 includes a locking mechanism 526, which is positioned on the base or handle 504 of the device 500. The device 500 includes first connecting means 518 similar to first connecting means 418 of the device 400 of FIG. 15. The device 500 further includes a first guiding member 544 in the form of open end 512 of first arm 506 of the body 502 of the device 500. The device 500 further includes second guiding means 546 in the form of a pulley. The device 500 further includes second connecting means 522 similar to second connecting means 422 of the device 400 of FIG. 15.

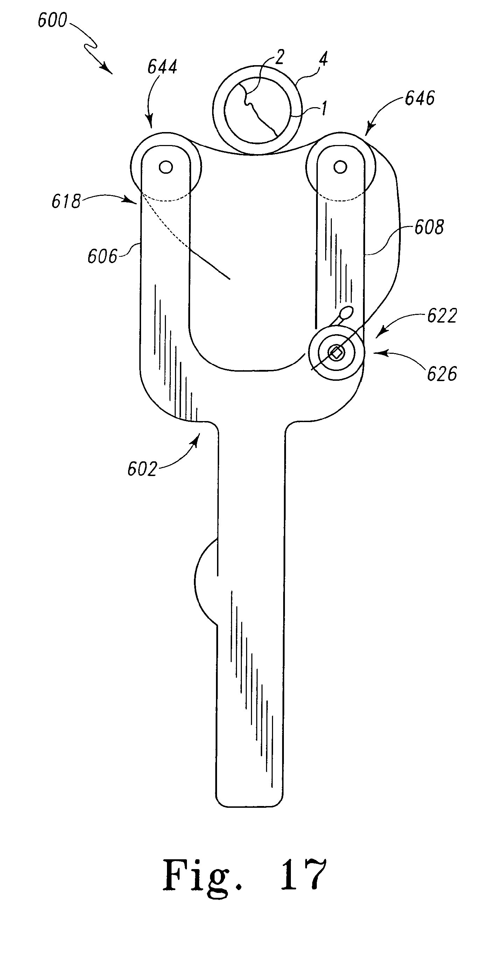

Referring now to FIG. 17, yet another embodiment of the present invention is shown as device 600. The device 600 includes a body 602 having a first arm 606 and a spaced apart second arm 608. The device 600 includes first connecting means 618 similar to first connecting means 518 of the device 500 of FIG. 16. The device 600 further includes first guiding means 644 in the form of a pulley.

The device 600 further includes second guiding means 646 in the form of a pulley. The device 600 further includes second connector means 622 similar to the second connecting means 522 of device 500 of FIG. 16. The device 600 further includes a locking mechanism 626 similar to the locking mechanism 526 of the device 500 of FIG. 16.

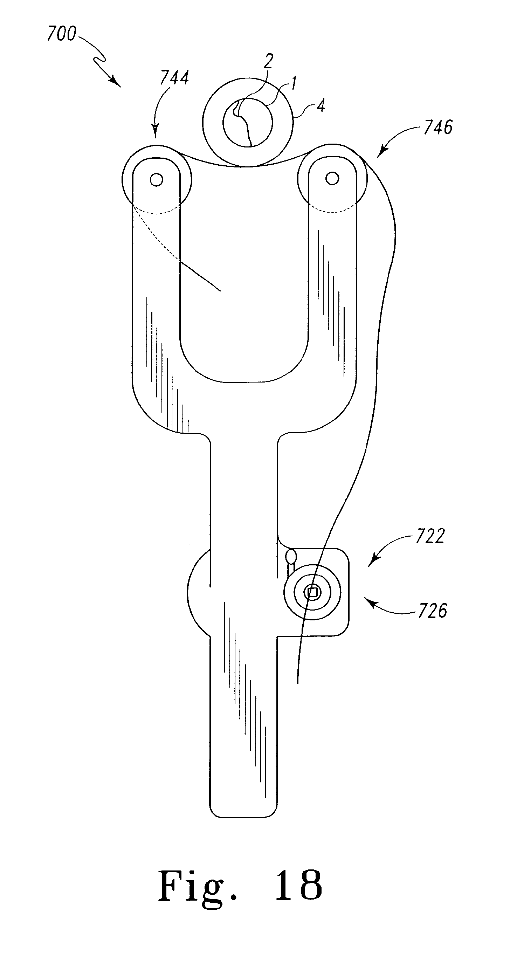

Referring now to FIG. 18, yet another embodiment of the present invention is shown as device first guide means 744, which is somewhat different than first guide means 544 of the device 500 of FIG. 16. In fact, first guide means 744 is similar to first guide means 644 of the device 600 of FIG. 17. The device 700 further includes a second guide means 746 similar to the second guide means 546 of the device 500 of FIG. 16. The device 700 further includes second connecting means 722 similar to second connecting means 522 of the device 500 of FIG. 16. The device 700 further includes a locking mechanism 726 similar to the locking mechanism 526 of the device 500 of FIG. 18.

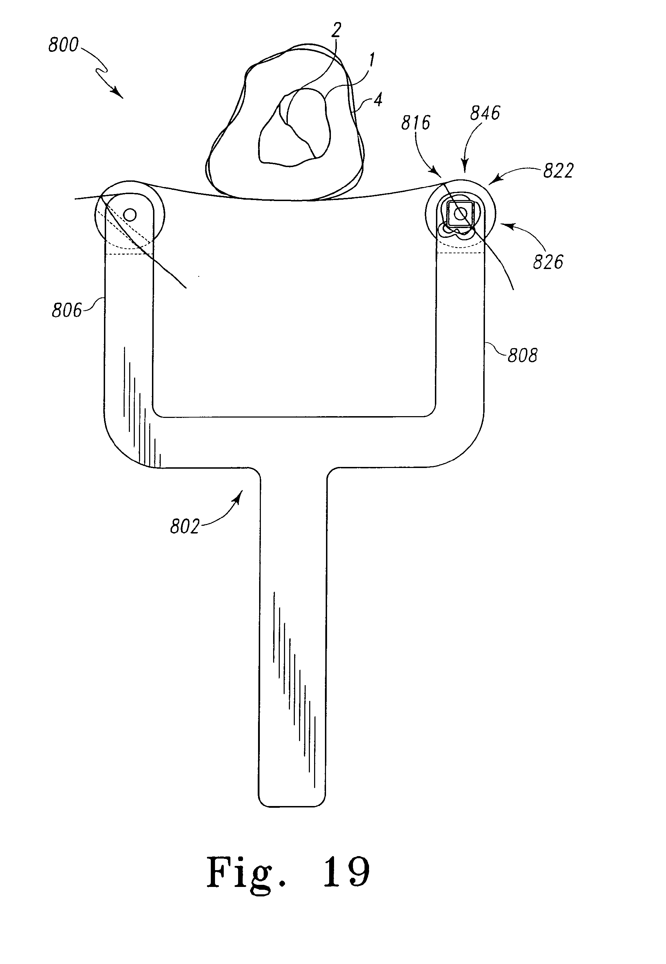

Referring now to FIG. 19, yet another embodiment of the present invention is shown as device 800. The device 800 includes a body 802, which has a shape somewhat different than that of the body 702 of the device 700 and the body 502 of the device 500 of FIGS. 17 and 16, respectively. For example, and as shown in FIG. 19, the body 802 includes a first arm 806, which is spaced at a greater distance from second arm 808 than that of either the device 700 of FIG. 18, or the device 500 of FIG. 16. The added space between the first arm 806 and the second arm 808 provides for use with a larger long bone or a long bone with a greater diameter. The device 800 further includes a locking mechanism 826 and second connecting means 822 that are positioned adjacent open end 816 of the second arm 808. The positioning of second connecting means 822 and locking mechanism 826 near the open end 816 of the second arm 808 provides for the shortest cable length, but requires an open procedure when installing the cable 4. Second connecting means 822 and the locking mechanism 826 are similar to second connecting means 722 and the locking mechanism 726, respectively, of the device 700 of FIG. 18.

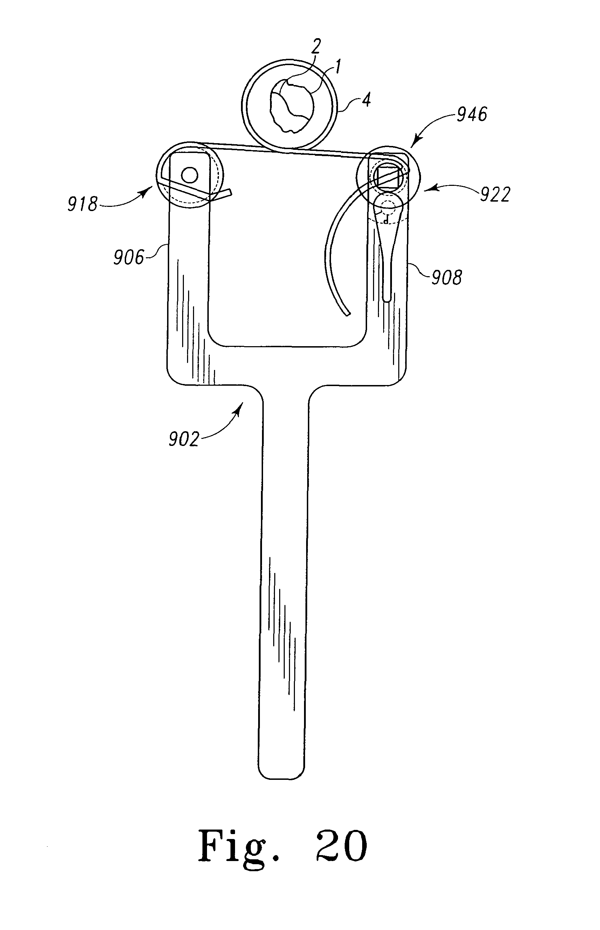

Referring now to FIG. 20, yet another embodiment of the present invention is shown as device 900. The device 900 is similar to the device 700 of FIG. 18 and includes a body 902 from which spaced apart first arm 906 and second arm 908 extend. The device 900 includes first connecting means 918 similar to first connecting means 218 of the device 200 of FIG. 4. The device 900 further includes a first guide member 944 similar to the first guide member 244 of the device 200 of FIG. 4. The device 900 further includes a second guide member 946, second connecting means 922 and a second locking mechanism 926 similar to the second member 846, second connecting means 822 and second locking mechanism 826, respectively, of the device 800 of FIG. 19.

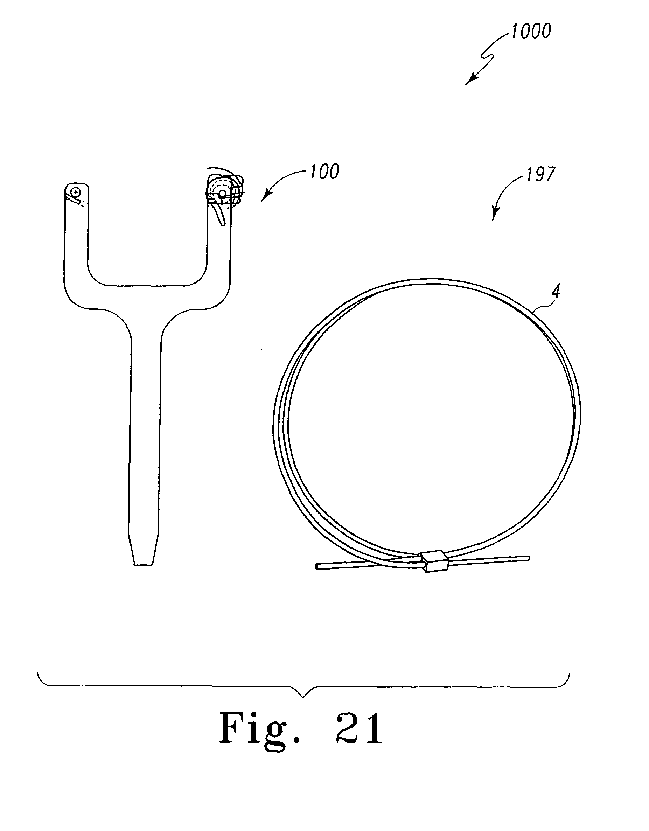

Referring now to FIG. 21, yet another embodiment of the present invention is shown as kit 1000. The kit 1000 includes the device 100 as well as cerclage cable system 197.

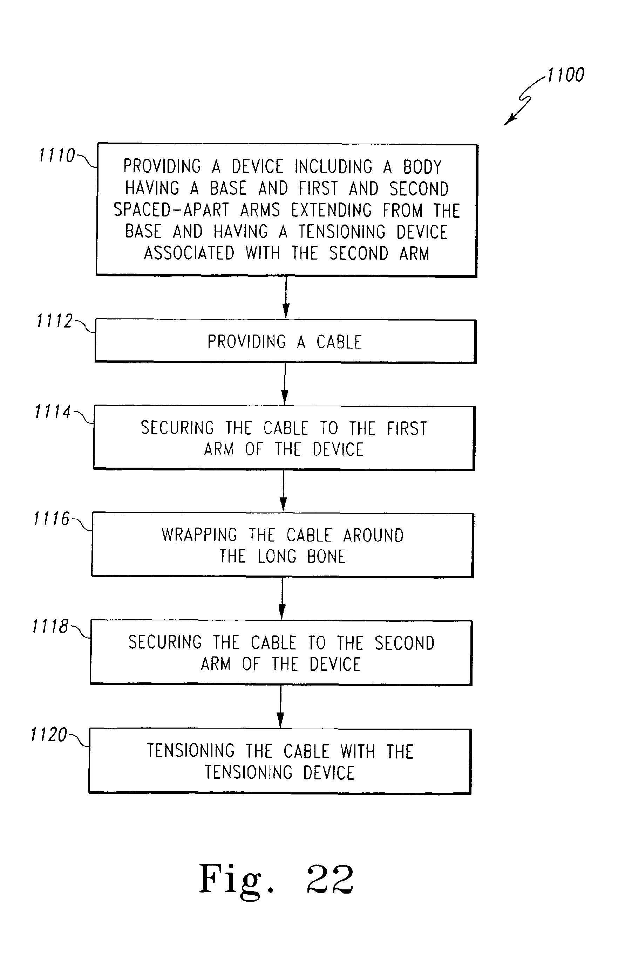

Referring now to FIG. 22, yet another embodiment of the present invention is shown as surgical procedure or method 1100. The method 1100 includes a first step 1110 of providing a device including a body having a base and first and second spaced-apart arms extending from the base and having a tensioning device connected to the second arm. The method 1100 further includes a second step 1112 of providing a cable and a third step 1114 of securing the cable to the first arm of the device. The method 1100 further includes a fourth step 1116 of wrapping the cable around the long bone and a fifth step 1118 of securing the cable to the second arm of the device. The method 1100 further includes a sixth step 1120 of tensioning the cable with the tensioning device

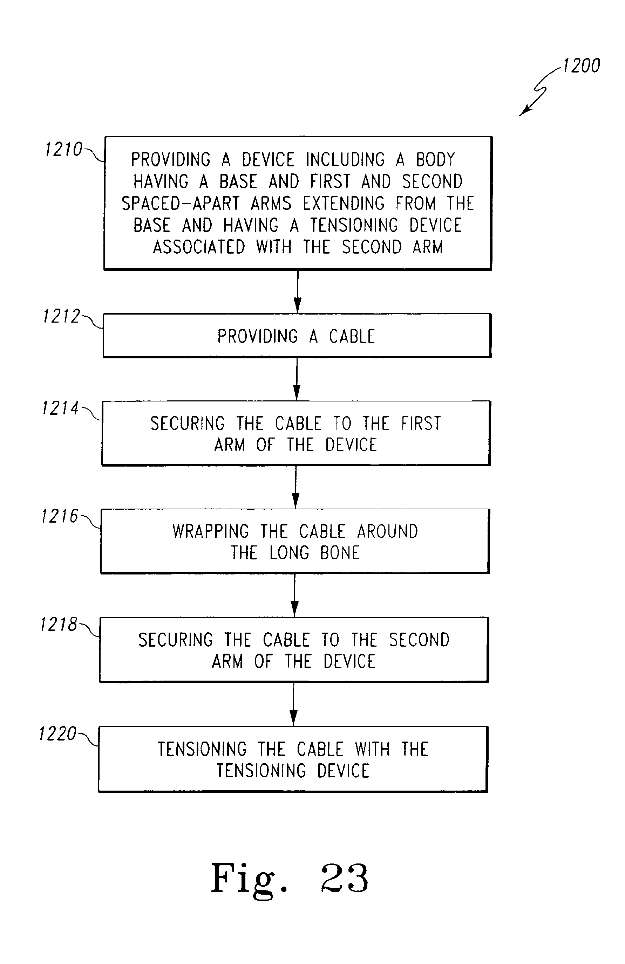

Referring now to FIG. 23, yet another embodiment of the present invention is shown as surgical procedure or method 1200. The method 1200 includes a first step 1210 of providing a device including a body having a base and first and second space apart arms extending from the base, the arms having proximal and distal ends of the arms and having a tensioning device associated with the second arm. The method 1200 further includes a second step 1212 of providing a cable and a third step 1214 of securing the cable to the distal end of the first arm of the device. The method 1200 further includes a fourth step 1216 of wrapping the cable around the long bone and a fifth step 1218 of securing the cable to the distal end of the second arm of the device. The method 1200 further includes a sixth step 1220 of tensioning the cable with the tensioning device.

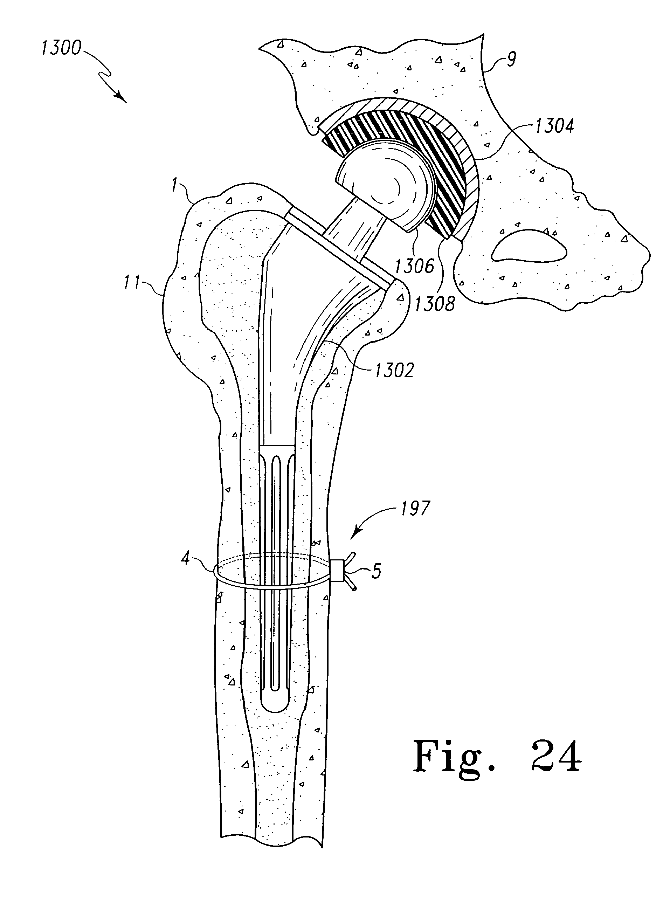

Referring now to FIG. 24, hip prosthesis 1300 is shown for use with the cerclage cable system 197 of FIG. 1Q. The hip prosthesis 1300 includes a hip stem 1302, which is secured in femur or long bone 1. The hip prosthesis 1300 further includes an acetabular shell 1304, which is secured to acetabulum 9. The hip prosthesis 1300 further includes a head 1306, which is connected to the hip stem 1302. The prosthesis 1300 may further include a liner 1308, which is positioned between the head 1306 and the shell 1304. It should be appreciated that the head 1306 may cooperate directly with the shell 1304. The insertion of a hip stem 1302 into a femur 1 may cause the femur 1 to fracture. This may be true with the use of a cement system or with a cementless system. The femur 1 may more likely fracture when installing a hip stem in a cementless procedure. If the insertion of the hip stem 1302 in the femur 1 causes the femur to fracture or if the femur 1 already has a fracture, the cerclage cable system 197 may be positioned around the cortical bone 11 of the femur. The cable 4 may be secured with the sleeve 5 around the femur 1.

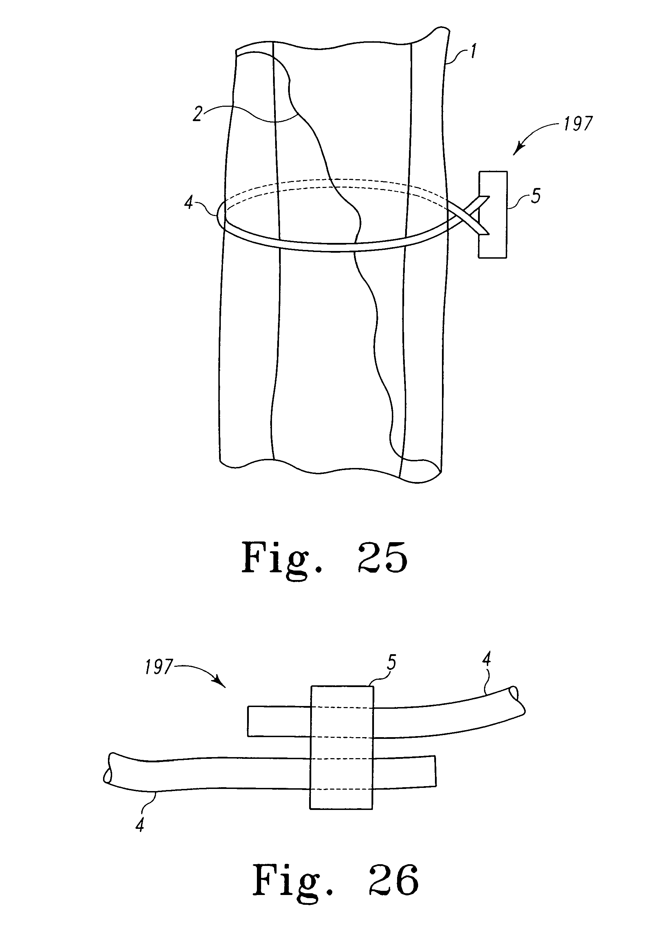

Referring now to FIG. 25, the cerclage cable system 197 is shown in position around femur 1 to secure fracture 2 formed in the femur 1. The cerclage cable system 197 includes the cable 4, which is secured by sleeve 5.

Referring now to FIG. 26, the connector or sleeve 5 is shown in greater detail with the cable 4 connected to the connector 5.

## Claims

1. A kit for use in orthopaedic procedures on a long bone, said kit comprising:
a cable; and
a device including a body and a first connecting means, said first connecting means operably associated with one of the body and the first arm for connecting a first end of the cable to the device, said device further including a second connecting means operably associated with one of the body and the second arm for connecting a second end of the cable to the device, at least one of said first connecting means and said second connecting means comprising a unidirectional locking mechanism for tightening the cable.

2. A kit as claimed in claim 1, wherein said body includes a base and first and second space apart arms extending from the base, said first and second arms each including a closed end attached to the base and an open end opposed to the closed end.

3. A kit as claimed in claim 2, wherein said first and second arms extend generally parallel to each other.

4. A kit as claimed in claim 1, wherein said unidirectional locking mechanism comprises a ratcheting mechanism.

5. A kit as claimed in claim 4, wherein said ratcheting mechanism comprises a pair of spaced apart wheels, each wheel having teeth formed thereon and biasing member to urge the teeth of wheels into engagement with each other, the wheels adapted for relative rotation therebetween in a first direction and for locked engagement in a second opposed direction.

6. A kit as claimed in claim 1, wherein said unidirectional locking mechanism comprises a cam locking mechanism.

7. A kit as claimed in claim 2, wherein said locking mechanism is positioned spaced from the open end of one of said first arm and said second arm.

8. A kit as claimed in claim 2, further comprising guiding means attached to the open end of at least one of said first arm and said second arm for guiding the cable.

9. A kit as claimed in claim 8, wherein said guiding means comprises a pulley attached to the open end of at least one of said first arm and said second arm for guiding the cable.

10. A kit as claimed in claim 8, wherein said guiding means comprises a groove formed in the open end of least one of said first arm and said second arm.

11. A kit as claimed in claim 2, wherein one of said one of said first connecting means and said second connecting means is operably associated with one of said first arm and said second arm.

12. A kit as claimed in claim 10, wherein one of said one of said first connecting means and said second connecting means includes a clamping mechanism attached to one of said first arm and said second arm.

13. A kit as claimed in claim 10, wherein one of said one of said first connecting means and said second connecting means is defined by an opening formed in one of said first arm and said second arm for slidably receiving the cable.

14. A device for use in tensioning a cable around a long bone for use in orthopaedic procedures, said device comprising:
a base;
a first spaced-apart arm extending from the base, said first arm including a closed end attached to the base and an open end opposed to the closed end;
a second spaced-apart arm extending from the base, said second arm including a closed end attached to the base and an open end opposed to the closed end, first connecting means, said first connecting means connected to said first arm for connecting a first end of the cable to the device;
second connecting means connected to said second arm for connecting a second end of the cable to the device, said first connecting means comprising a unidirectional locking mechanism for tightening the cable;
a body, said body including a base and first and second space apart arms extending from the base, said first and second arms each including a closed end attached to the base and an open end opposed to the closed end;
first connecting means operably associated with one of the body and the first arm for connecting a first end of the cable to the device; and
second connecting means operably associated with one of the body and the second arm for connecting a second end of the cable to the device.

15. A device as claimed in claim 14, wherein at least one of said first connecting means and said second connecting means comprising a unidirectional locking mechanism for tightening the cable.

16. A device as claimed in claim 15, wherein said unidirectional locking mechanism comprises a ratcheting mechanism.

17. A device as claimed in claim 16, wherein said ratcheting mechanism comprises a pair of spaced apart wheels, each wheel having teeth formed thereon and biassing member to urge the teeth of wheels into engagement with each other, the wheels adapted for relative rotation between them in a first direction and for locked engagement in a second opposed direction.
